# EUROPEAN PATENT SPECIFICATION

(11) **EP 4 076 465 B1**
(45) Date of publication and mention of the grant of the patent: **15.11.2023**
(21) Application number: 20828324.2
(22) Date of filing: 15.12.2020
(51) Int. Cl.: A61K 31/519, A61K 31/337, A61P 35/00, A61K 9/00, A61K 39/00, A61K 9/20

(54) **ONCE DAILY CANCER TREATMENT REGIMEN WITH A PRMT5 INHIBITOR**
EINMAL TÄGLICHES KREBSBEHANDLUNGSREGIME MIT EINEM PRMT5-INHIBITOR
SCHÉMA DE TRAITEMENT D'UN CANCER UNE FOIS PAR JOUR AVEC UN INHIBITEUR DE PRMT5

(30) Priority: 18.12.2019 US 201962949688 P; 17.08.2020 US 202063066755 P; 02.11.2020 US 202063108745 P
(43) Date of publication of application: 26.10.2022
(73) Proprietor: Pfizer Inc., New York, NY 10001-2192 (US)
(72) Inventor: LI, Meng, San Diego, California 92121 (US); LIAO, Kai-Hsin, San Diego, California 92121 (US); YAMAZAKI, Shinji, San Diego, California 92130 (US)
(74) Representative: Pfizer
(86) International application number: PCT/IB2020/061957
(87) International publication number: WO 2021/124096

(56) References cited:
- WO-A1-2020/152557
- US-B2- 10 220 037
- Pfizer: "A Dose Escalation Study Of PF-06939999 In Participants With Advanced Or Metastatic Solid Tumors", ClinicalTrials.gov Identifier: NCT03854227, 26 February 2019 (2019-02-26), XP055775381, Retrieved from the Internet: URL:https://www.clinicaltrials.gov/ct2/sho w/NCT03854227 [retrieved on 2021-02-12] cited in the application

## Description

### Field of the Invention

The present invention is directed to once-daily administration of the PRMT5 inhibitor: (1S,2S,3S,5R)-3-((6-(difluoromethyl)-5-fluoro-1,2,3,4-tetrahydroisoquinolin-8-yl)oxy)-5-(4-methyl-7H-pyrrolo[2,3-d]pyrimidin-7-yl)cyclopentane-1,2-diol (Compound 1), having the structure: 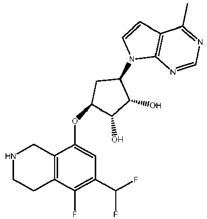 or a pharmaceutically acceptable salt thereof to treat cancers, either alone as a monotherapy or in combination with one or more therapeutic agents.

### Background of the Invention

U.S. Patent No. 10,220,037 discloses various compounds and compositions known to inhibit PRMT5 and treat various cancer indications. Among these compounds described in U.S. Patent No. 10,220,037 is (1S,2S,3S,5R)-3-((6-(difluoromethyl)-5-fluoro-1,2,3,4-tetrahydroisoquinolin-8-yl)oxy)-5-(4-methyl-7H-pyrrolo[2,3-d]pyrimidin-7-yl)cyclopentane-1,2-diol: 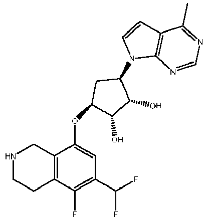 (Compound 1) and pharmaceutically acceptable salt thereof.

Pre-clinical data assessing the PK profile of Compound 1 demonstrated acceptable predicted PK profiles in humans. Briefly, in vivo plasma clearance (CL) in humans was predicted to be -3 and ~0.6 mL/min/kg from human liver microsomes and hepatocytes, respectively. The conservative value of ~3 mL/min/kg from human liver microsomes was used as the predicted CL. A steady-state volume of distribution (Vss) in humans was predicted to be 1.6 Ukg based on the mean unbound Vss scaling from rats and dogs factoring in human plasma protein binding. A half-life (t1/2) is predicted to be ~6 h calculated by the equation: t1/2 = 0.693*Vss/CL.

Moreover, based on the ~6-hour projected half-life for Compound 1 the likely peak to trough ratio in humans was predicted to be lower following twice daily (BID) dosing (~4) compared to once daily (QD) dosing (~8 or more). Lower peak to trough ratios are generally more desirable because they may help to minimize potential Cₘₐₓ-related adverse effects (if any) while maintaining the therapeutic effects.

These calculations, and in particular the half-life projection, were incorporated into the design of a Phase I clinical trial of Compound 1 [Study NCT03854227; A Phase 1 Study to Evaluate the Safety, Pharmacokinetics, and Pharmacodynamics of Escalating Doses Of [Compound 1] (PRMT Inhibitor) in Participants with Advanced or Metastatic Non-Small Cell Lung Cancer, Head And Neck Squamous Cell Carcinoma, Esophageal Cancer, Endometrial Cancer, Cervical Cancer And Bladder Cancer; first posted 26 FEB 2019] which included BID (twice daily) dosing of 0.5 mg, 1 mg, 2 mg, 4 mg, 8 mg, 16 mg, 30 mg and 60 mg Compound 1. BID (twice daily) dosing was implemented for all doses thought to potentially be therapeutic in order to limit (i.e., lessen) the projected peak to trough ratio of Compound 1 in view of the predicted ~6-hour half-life of Compound 1.

The present BID (twice daily) dosing scheme for Compound 1 is well tolerated, but patient convenience and compliance would be much improved if a once-daily regimen were possible. In this regard, it is noted that the primary indications for Compound 1 are various cancer types which are often accompanied by patients' physical and emotional difficulties in executing self-care. Moreover, many cancer indications become more prevalent with advancing age and is often accompanied by decline in memory. A once-daily regimen would be especially useful in enhancing compliance and convenience among these patients and their health care providers.

PRMT5 (Protein Arginine Methyltransferase 5) protein is found in both the nucleus and cytoplasm, and has multiple protein substrates such as histones, transcription factors and spliceosome proteins. PRMT5 has a binding partner, Mep50 (methylosome protein 50) and functions in multiple protein complexes. PRMT5 is associated with chromatin remodeling complexes (SWI/SNF, NuRD) and epigenetically controls genes involved in development, cell proliferation, and differentiation, including tumor suppressors, through methylation of histones (Karkhanis, V. et al., Versatility of PRMT5 Induced Methylation in Growth Control and Development, Trends Biochem Sci 36(12) 633-641 (2011)). PRMT5 also controls gene expression through association with protein complexes that recruit PRMT5 to methylate several transcription factors p53 (Jansson, M. et al., Arginine Methylation Regulates the p53 Response, Nat. Cell Biol. 10, 1431-1439 (2008)); E2F1 (Zheng, S. et al., Arginine Methylation-Dependent Reader-Writer Interplay Governs Growth Control by E2F-1, Mol Cell 52(1), 37-51 (2013)); HOXA9 (Bandyopadhyay, S. et al., HOXA9 Methylation by PRMT5 is Essential for Endothelial Cell Expression of Leukocyte Adhesion Molecules, Mol. Cell. Biol. 32(7):1202-1213 (2012)); and NFκB (Wei, H. et al., PRMT5 dimethylates R30 of the p65 Subunit to Activate NFκB, PNAS 110(33), 13516-13521 (2013)). In the cytoplasm, PRMT5 has a diverse set of substrates involved in other cellular functions including RNA splicing (Sm proteins), golgi assembly (gm130), ribosome biogenesis (RPS10), piRNA mediated gene silencing (Piwi proteins) and EGFR signaling (Karkhanis, 2011).

PRMT5 is overexpressed in many cancers and has been observed in patient samples and cell lines including B-cell lymphoma and leukemia (Wang, 2008) and the following solid tumors: gastric (Kim 2005) esophageal (Aggarwal, 2010), breast (Powers, 2011), lung (Gu, 2012), prostate (Gu, 2012), melanoma (Nicholas 2012), colon (Cho, 2012) and ovarian (Bao, 2013). In many of these cancers, overexpression of PRMT5 correlated with poor prognosis.

The references to methods of treatment in the summary and detailed description of the invention in this description are to be interpreted as references to the compounds, pharmaceutical compositions and medicaments of the present invention for use in a method for treatment of the human (or animal) body by therapy.

### Summary of the Invention

The present invention resides in the surprising discovery that QD (once daily) dosing is possible with Compound 1, and that QD dosing would result in substantially lower peak to trough ratios in patients than had previously been predicted based on pre-clinical data.

Additionally, the invention resides in the discovery of a single dosage form of Compound 1 designed to be dosed on a QD schedule.

Accordingly, in one aspect, the invention provides a method of treating abnormal cell growth in a subject, said method comprising administration to said subject of a therapeutically effective amount of (1S,2S,3S,5R)-3-((6-(difluoromethyl)-5-fluoro-1,2,3,4-tetrahydroisoquinolin-8-yl)oxy)-5-(4-methyl-7H-pyrrolo[2,3-d]pyrimidin-7-yl)cyclopentane-1,2-diol (Compound 1) having the structure: 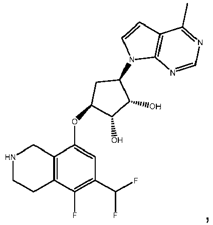 or a pharmaceutically acceptable salt thereof, no more than once per day (e.g., once a day).

In some embodiments, the abnormal cell growth is treated by inhibiting the activity of PRMT5 proteins in said subject. In some embodiments, the abnormal cell growth is cancer. In some embodiments, the cancer is selected from the group consisting of lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, and pituitary adenoma. In some embodiments, the cancer is non-small cell lung cancer (NSCLC), head and neck squamous cell carcinoma (HNSCC), esophageal cancer, endometrial cancer, cervical cancer, bladder cancer, urothelial carcinoma, or pancreatic cancer.

In some embodiments, Compound 1, or a pharmaceutically acceptable salt thereof, is administered in a dosage form selected from one or more tablets, one or more capsules, a liquid solution, a liquid suspension, or a syrup. In some embodiments, the administration of Compound 1 takes place for seven consecutive days.

In some embodiments, the administration takes place for one cycle comprising twenty-eight consecutive days. In some embodiments, the administration takes place for 1-24 cycles, such as 4 cycles, 6 cycles, 12 cycles, or 24 cycles.

In some embodiments, Compound 1 is administered to a subject at a dose of about 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 50 mg, 60 mg, 70 mg, 80 mg, 90 mg, 100 mg, 110 mg, 120 mg, 130 mg, 140 mg, or 150 mg once daily.

In another aspects, the invention further provides a method of treating abnormal cell growth in a subject, said method comprising oral administration to said subject of a therapeutically effective amount of (1S,2S,3S,5R)-3-((6-(difluoromethyl)-5-fluoro-1,2,3,4-tetrahydroisoquinolin-8-yl)oxy)-5-(4-methyl-7H-pyrrolo[2,3-d]pyrimidin-7-yl)cyclopentane-1,2-diol (Compound 1) having the structure: 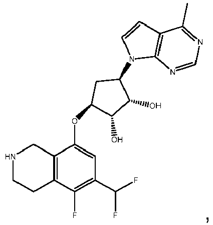 or a pharmaceutically acceptable salt thereof, no more than once per day (e.g., once a day), and administration of an effective amount of a second or more therapeutic agents. In some embodiments, the second therapeutic agent is a chemotherapeutic agent (e.g., paclitaxel or docetaxel).

In some embodiments, the second therapeutic agent is administered intravenously, subcutaneously, or orally. In some embodiments, the second therapeutic agent is administered to the subject simultaneously, concurrently, or sequentially with Compound 1.

In some embodiments, the second therapeutic agent is docetaxel being administered at 75 mg/m² intravenously once every 21 days (e.g., administration over 1 hour on day 1 of a cycle comprising 21 days).

In some embodiments, the second therapeutic agent is a biotherapeutic agent such as an antibody. In some embodiments, the antibody is a PD-1 or PD-L1 antibody.

In some embodiments, the subject treated with the treatment methods described herein is premedicated with a corticosteroid prior to the treatment.

In some embodiments, the cancer treated with the treatment methods described herein is relapsed, refractory, or metastatic.

In another aspect, the invention further provides a medicament (also referred to herein as a pharmaceutical composition) comprising a therapeutically effective amount of (1S,2S,3S,5R)-3-((6-(difluoromethyl)-5-fluoro-1,2,3,4-tetrahydroisoquinolin-8-yl)oxy)-5-(4-methyl-7H-pyrrolo[2,3-d]pyrimidin-7-yl)cyclopentane-1,2-diol (Compound 1) having the structure: 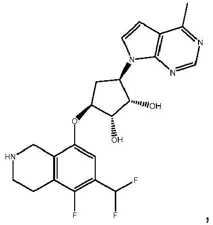 or a pharmaceutically acceptable salt thereof, for use in treating abnormal cell growth in a subject no more than once per day (e.g., once a day).

In another aspect, the invention further provides a medicament (also referred to herein as a pharmaceutical composition) comprising a therapeutically effective amount of (1S,2S,3S,5R)-3-((6-(difluoromethyl)-5-fluoro-1,2,3,4-tetrahydroisoquinolin-8-yl)oxy)-5-(4-methyl-7H-pyrrolo[2,3-d]pyrimidin-7-yl)cyclopentane-1,2-diol (Compound 1) having the structure: 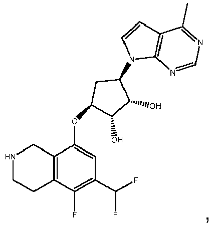 or a pharmaceutically acceptable salt thereof, and an effective amount of a second therapeutic agent for use in treating abnormal cell growth in a subject no more than once per day (e.g, once a day).

### Detailed Description of the Drawings

**FIG. 1** depicts day 1 median PK profiles for Compound 1 dosed BID at 2 mg.
**FIG. 2** depicts "steady state" (day 15) median PK profiles for Compound 1 dosed BID at 2 mg.
**FIG. 3** depicts the simulated steady state PK profiles (QD and BID) described in Example 1.
**FIG. 4** depicts the clinical study schema for monotherapy and combination therapy using Compound 1.
**FIG. 5** depicts steady state (day 15) median PK profiles for Compound 1 dosed QD at 4 mg.
**FIG. 6** depicts steady state (day 15) median PK profiles for Compound 1 dosed QD at 6 mg.
**FIG. 7** depicts steady state (day 15) median PK profiles for Compound 1 dosed QD at 8 mg.
**FIG. 8** depicts blood pharmacodynamic (PD) marker SDMA (Symmetrical Dimethylarginine) inhibition with Compound 1.

### Detailed Description of the Invention

The invention disclosed herein is directed to methods of treating abnormal cell growth (e.g., cancer) in a subject comprising administering a therapeutically effective amount of a PRMT5 inhibitor. Accordingly, in one aspect, the invention provides method of treating abnormal cell growth in a subject, said method comprising (e.g., oral) administration to said subject of a therapeutically effective amount of (1S,2S,3S,5R)-3-((6-(difluoromethyl)-5-fluoro-1,2,3,4-tetrahydroisoquinolin-8-yl)oxy)-5-(4-methyl-7H-pyrrolo[2,3-d]pyrimidin-7-yl)cyclopentane-1,2-diol (Compound 1) having the structure: 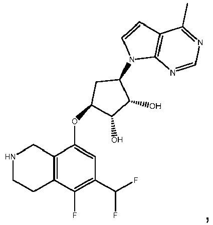 or a pharmaceutically acceptable salt thereof, no more than once per day (e.g., once a day).

In some embodiments, the method described herein further comprises administering an effective amount of a second or more therapeutic agent, such as a chemotherapeutic agent or a biotherapeutic agent.

### Definitions

The present invention may be understood more readily by reference to the following detailed description of the preferred embodiments of the invention and the Examples included herein. It is to be understood that the terminology used herein is for the purpose of describing specific embodiments only and is not intended to be limiting. It is further to be understood that unless specifically defined herein, the terminology used herein is to be given its traditional meaning as known in the relevant art.

As used herein, the singular form "a", "an", and "the" include plural references unless indicated otherwise. For example, "a" substituent includes one or more substituents.

The term "about" which is used to modify a numerically defined parameter means that the parameter may vary by as much as 10% above or below the stated numerical value for that parameter, i.e. ± 10%. For example, a dose of "about 5 mg/kg" should be understood to mean "5 mg/kg ± 10%", i.e. the dose may vary between 4.5 mg/kg and 5.5 mg/kg, unless otherwise specified.

The term "patient" or "subject" refer to any single subject for which therapy is desired or that is participating in a clinical trial, epidemiological study or used as a control, including humans and mammalian veterinary patients such as cattle, horses, dogs and cats. In certain preferred embodiments, the subject is a human.

The term "treat" or "treating" cancer or an associated condition or disease as used herein means to administer a therapy according to the present invention to a subject having cancer or an associated condition or disease to achieve at least one positive therapeutic effect. The term "treatment", as used herein, unless otherwise indicated, refers to the act of treating as "treating" is defined immediately above. The term "treating" also includes adjuvant and neo-adjuvant treatment of a subject.

As used in herein, "administering" refers to the delivery of a therapeutic agent to a subject, using any of the various methods and delivery systems known to those skilled in the art. Exemplary routes of administration include intravenous, intramuscular, subcutaneous, intraperitoneal, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion, as well as in vivo electroporation. A therapeutic agent can be administered via a non-parenteral route, or orally. Other non-parenteral routes include a topical, epidermal or mucosal route of administration, for example, intranasally, vaginally, rectally, sublingually or topically. Administering can also be performed, for example, once, a plurality of times, and/or over one or more extended periods.

The term "BID" (or "bid" or "b.i.d.") means medication is administered twice (two times) a day. For instance, 8 mg BID represents a daily dose of 16 mg administered in a first and second daily 8 mg dose.

The term "QD" (or "qd" or "q.d.") means medication is administered once (one time) a day

The terms "treatment regimen", "dosing protocol" and "dosing regimen" are used interchangeably to refer to the dose and timing of administration of each therapeutic agent in a combination of the invention.

"Ameliorating" means a lessening or improvement of one or more symptoms upon treatment with a combination described herein, as compared to not administering the combination. "Ameliorating" also includes shortening or reduction in duration of a symptom.

As used herein, an "effective dosage" or "effective amount" of drug, compound or pharmaceutical composition is an amount sufficient to effect any one or more beneficial or desired, including biochemical, histological and / or behavioural symptoms, of the disease, its complications and intermediate pathological phenotypes presenting during development of the disease. For therapeutic use, a "therapeutically effective amount" refers to that amount of a compound being administered which will relieve to some extent one or more of the symptoms of the disorder being treated. An effective dosage can be administered in one or more administrations. For the purposes of this invention, an effective dosage of drug, compound, or pharmaceutical composition is an amount sufficient to accomplish prophylactic or therapeutic treatment either directly or indirectly. As is understood in the clinical context, an effective dosage of drug, compound or pharmaceutical composition may or may not be achieved in conjunction with another drug, compound or pharmaceutical composition.

Pharmaceutically acceptable refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability.

Peak to trough ratio refers to the ratio of the maximum concentration (Cₘₐₓ) to minimum concentration (Cₘᵢₙ) of Compound 1 observed. Relevant to the present invention is the peak to trough ration during the dosing interval at "steady state" (day 15).

Half-life, for instance the half-life of Compound 1, refers to the time it takes for the plasma concentration of Compound 1 to decrease by 50% as calculated by the equation: 0.693* Vss/CL where Vss is the steady-state volume of distribution and CL is plasma clearance.

"Chemotherapeutic agent" is a chemical compound useful in the treatment of cancer and/or cancer-associated disease. Classes of chemotherapeutic agents include, but are not limited to: alkylating agents, antimetabolites, kinase inhibitors, spindle poison plant alkaloids, cytotoxic/antitumor antibiotics, topoisomerase inhibitors, photosensitizers, anti-estrogens and selective estrogen receptor modulators (SERMs), anti-progesterones, estrogen receptor down-regulators (ERDs), estrogen receptor antagonists, leutinizing hormone-releasing hormone agonists, anti-androgens, aromatase inhibitors, EGFR inhibitors, VEGF inhibitors, and anti-sense oligonucleotides that inhibit expression of genes implicated in abnormal cell proliferation or tumor growth. Chemotherapeutic agents are further described herein.

"Chemotherapy" as used herein, refers to a chemotherapeutic agent, as defined above, or a combination of two, three or four chemotherapeutic agents, for the treatment of cancer and/or cancer-associated disease. When chemotherapy consists more than one chemotherapeutic agent, the chemotherapeutic agents can be administered to the patient on the same day or on different days in the same treatment cycle.

Exemplary methods and materials are described herein, although methods and materials similar or equivalent to those described herein can also be used in the practice or testing of the present invention. The materials, methods, and examples are illustrative only and not intended to be limiting

### Methods, Uses, and Medicaments

The treatment methods of the present invention are directed to the administration of a PRMT 5 inhibitor, and pharmaceutically acceptable salts thereof, to a subject or subjects in need thereof, to treat abnormal cell growths such as cancers. These cancers are typically solid tumor cancers, for instance, pancreatic cancer, endometrial cancer, non-small cell lung cancer (NSCLC), endometrial cancer, cervical cancer, bladder cancer, esophageal cancer, head and neck squamous cell carcinoma (HNSCC), and other cancers.

Thus there is provided embodiments of the invention including a method of treating abnormal cell growth in a subject, said method comprising oral administration to said subject of a therapeutically effective amount of (1S,2S,3S,5R)-3-((6-(difluoromethyl)-5-fluoro-1,2,3,4-tetrahydroisoquinolin-8-yl)oxy)-5-(4-methyl-7H-pyrrolo[2,3-d]pyrimidin-7-yl)cyclopentane-1,2-diol (Compound 1) having the structure: 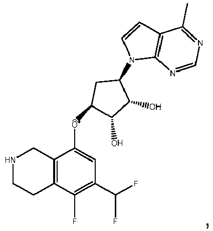 or a pharmaceutically acceptable salt thereof, no more than once per day (e.g., once a day).

Pharmaceutically acceptable salts of Compound 1 include the acetate, aspartate, benzoate, besylate, bicarbonate/carbonate, bisulphate/sulfate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate, aluminum, arginine, benzathine, calcium, choline, diethylamine, diolamine, glycine, lysine, magnesium, meglumine, olamine, potassium, sodium, tromethamine, tosylate, and zinc salts. For a review on suitable salts, see "Handbook of Pharmaceutical Salts: Properties, Selection, and Use" by Stahl and Wermuth (Wiley-VCH, Weinheim, Germany, 2002).

In some embodiments of the invention, the abnormal cell growth is treated by inhibiting the activity of PRMT5 proteins in said subject. In some embodiments, the abnormal cell growth is cancer selected from the group consisting of lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, and pituitary adenoma.

In other embodiments, indications treated by the inventive methods include cancers selected from non-small cell lung cancer (NSCLC), head and neck squamous cell carcinoma (HNSCC), esophageal cancer, endometrial cancer, cervical cancer, bladder cancer, urothelial carcinoma, or pancreatic cancer.

In another aspect, provided is a method of treating abnormal cell growth (e.g., cancer) in a subject, said method comprising oral administration to said subject of a therapeutically effective amount of (1S,2S,3S,5R)-3-((6-(difluoromethyl)-5-fluoro-1,2,3,4-tetrahydroisoquinolin-8-yl)oxy)-5-(4-methyl-7H-pyrrolo[2,3-d]pyrimidin-7-yl)cyclopentane-1,2-diol (Compound 1) having the structure: 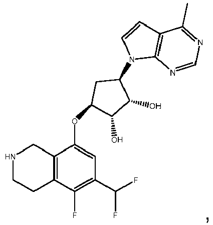 or a pharmaceutically acceptable salt thereof, no more than once per day (e.g., once a day), and further comprising administering an effective amount of a second therapeutic agent.

In some embodiments, the second therapeutic agent may comprise one or more of a chemotherapeutic agents, a biotherapeutic agent, an immunomodulating agent, a proteasome inhibitor, and a corticosteroid. Further therapeutic agents for use in the combination therapy of the present invention include a cancer vaccine, immune cell therapy (e.g. CAR-T cell-based therapy), radiotherapy, a vaccine, a cytokine therapy (e.g., immunostimulatory cytokines including various signaling proteins that stimulate immune response, such as interferons, interleukins, and hematopoietic growth factors), a targeted cytokine, an inhibitor of other immunosuppressive pathways, an inhibitors of angiogenesis, a T cell activator, an inhibitor of a metabolic pathway, an mTOR (mechanistic target of rapamycin) inhibitor (e.g., rapamycin, rapamycin derivatives, sirolimus, temsirolimus, everolimus, and deforolimus), an inhibitor of an adenosine pathway, a gamma secretase inhibitor (e.g. nirogacestat), a tyrosine kinase inhibitor including but not limited to INLYTA^{®}, ALK (anaplastic lymphoma kinase) inhibitors (e.g., crizotinib, ceritinib, alectinib, and sunitinib), a BRAF inhibitor (e.g., vemurafenib and dabrafenib), a PI3K inhibitor, a HPK1 inhibitor, an epigenetic modifier, an inhibitors or depletor of Treg cells and/or of myeloid-derived suppressor cells, a JAK (Janus Kinase) inhibitor (e.g., ruxolitinib and tofacitinb, varicitinib, filgotinib, gandotinib, lestaurtinib, momelotinib, pacritinib, and upadacitinib), a STAT (Signal Transducers and Activators of Transcription) inhibitor (e.g., STAT1, STAT3, and STAT5 inhibitors such as fludarabine), a cyclin-dependent kinase (CDK) or other cell cycle inhibitor, an immunogenic agent (for example, attenuated cancerous cells, tumor antigens, antigen presenting cells such as dendritic cells pulsed with tumor derived antigen or nucleic acids, a MEK inhibitor (e.g., trametinib, cobimetinib, binimetinib, and selumetinib), a GLS1 inhibitor, a PARP inhibitor (e.g. talazoparib, olaparib, rucaparib, niraparib) , an oncolytic virus, gene therapies including DNA, RNA delivered directly or by adeno-associated viruses (AAV) or nanoparticles, an innate immune response modulator (e.g., TLRs, KIR, NKG2A), an IDO (Indoleamine-pyrrole 2,3-dioxygenase) inhibitor, a PRR (Pattern Recognition Receptors) agonist, and cells transfected with genes encoding immune stimulating cytokines such as but not limited to GM-CSF).

The treatment methods described herein may comprise administration of one or more chemotherapeutic agents. Examples of chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin gamma1l and calicheamicin phil1, see, e.g., Agnew, Chem. Intl. Ed. Engl., 33:183-186 (1994); dynemicin, including dynemicin A; bisphosphonates, such as clodronate; an esperamicin; as well as neocarzinostatin chromophore and related chromoprotein enediyne antibiotic chromomophores), aclacinomysins, actinomycin, authramycin, azaserine, bleomycins, cactinomycin, carabicin, caminomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; FOLFOX including folinic acid, 5-FU and oxaliplatin; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; razoxane; rhizoxin; sizofuran; spirogermanium; tenuazonic acid; triaziquone; 2, 2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel and docetaxel; chlorambucil; gemcitabine; 6-thioguanine; mercaptopurine; methotrexate; platinum analogs such as carboplatin; cisplatin; vinblastine; platinum; etoposide (VP-16); ifosfamide; mitoxantrone; vincristine; vinorelbine; novantrone; teniposide; edatrexate; daunomycin; aminopterin; xeloda; ibandronate; CPT-11; topoisomerase inhibitor RFS 2000; difluoromethylornithine (DMFO); retinoids such as retinoic acid; capecitabine; and pharmaceutically acceptable salts, acids or derivatives of any of the above.

The methods described herein may comprise administering one or more biotherapeutic agents such as antibodies. Examples of antibodies including, but not limited to, an anti-CTLA-4 antibody, an anti-CD3 antibody, an anti-CD4 antibody, an anti-CD8 antibody, an anti-4-1 BB antibody, an anti-PD-1 antibody, an anti-PD-L1 antibody, an anti-TIM3 antibody, an anti-LAG3 antibody, an anti-TIGIT antibody, an anti-OX40 antibody, an anti-IL-7Ralpha (CD127) antibody, an anti-IL-8 antibody, an anti-IL-15 antibody, an anti-HVEM antibody, an anti-BTLA antibody, an anti-CD38 antibody, an anti-CD40 antibody, an anti-CD40L antibody, anti-CD47 antibody, an anti-CSF1R antibody, an anti-CSF1 antibody, an anti-IL-7R antibody, an anti-MARCO antibody, an anti-CXCR4 antibodies, an anti-VEGF antibody, an anti-VEGFR1 antibody, an anti-VEGFR2 antibody, an anti-TNFR1 antibody, an anti-TNFR2 antibody, an anti-CD3 bispecific antibody, an anti-CD19 antibody, an anti-CD20, an anti-Her2 antibody, an anti-EGFR antibody, an anti-ICOS antibody, an anti-CD22 antibody, an anti-CD52 antibody, an anti-CCR4 antibody, an anti-CCR8 antibody, an anti-CD200R antibody, an anti-VISG4 antibody, an anti-CCR2 antibody, an anti-LlLRb2 antibody, an anti-CXCR4 antibody, an anti-CD206 antibody, an anti-CD163 antibody, an anti-KLRG1 antibody, an anti-FLT3 antibody, an anti-B7-H4 antibody, an anti-B7-H3 antibody, an KLRG1 antibody, a BTN1A1 antibody, a BCMA antibody, an anti-SLAMF7 antibody, an anti-avb8 antibody, an anti-CD80 antibody, or an anti-GITR antibody.

In some embodiments, the second therapeutic agent is an anti-PD-1 antibody or an anti-PD-L1 antibody. Examples of anti-PD-1 and anti-PD-L1 antibodies include, but are not limited to, atezolizumab (TECENTRIQO, MPDL3280A, Roche Holding AG), durvalumab (IMFINZIO, AstraZeneca PLC), nivolumab (OPDIVO^{®}, ONO-4538, BMS-936558, MDX1106, Bristol-Myers Squibb Company), pembrolizumab (KEYTRUDA^{®}, MK-3475, lambrolizumab, Merck & Co., Inc.), BCD-100 (BIOCAD Biopharmaceutical Company), tislelizumab (BGB-A317, BeiGene Ltd./Celgene Corporation), genolimzumab (CBT-501, CBT Pharmaceuticals), CBT-502 (CBT Pharmaceuticals), GLS-010 (Harbin Gloria Pharmaceuticals Co., Ltd.), sintilimab (IBI308, Innovent Biologics, Inc.), WBP3155 (CStone Pharmaceuticals Co., Ltd.), AMP-224 (GlaxoSmithKline plc), BI 754091 (Boehringer Ingelheim GmbH), BMS-936559 (Bristol-Myers Squibb Company), CA-170 (Aurigene Discovery Technologies), FAZ053 (Novartis AG), spartalizumab (PDR001, Novartis AG), LY3300054 (Eli Lilly & Company), MEDI0680 (AstraZeneca PLC), PDR001 (Novartis AG), sasanlimab (PF-06801591, Pfizer Inc.), cemiplimab (LIBTAYO^{®}, REGN2810, Regeneron Pharmaceuticals, Inc.), camrelizumab (SHR-1210, Incyte Corporation), TSR-042 (Tesaro, Inc.), AGEN2034 (Agenus Inc.), CX-072 (CytomX Therapeutics, Inc.), JNJ-63723283 (Johnson & Johnson), MGD013 (MacroGenics, Inc.), AN-2005 (Adlai Nortye), ANA011 (AnaptysBio, Inc.), ANB011 (AnaptysBio, Inc.), AUNP-12 (Pierre Fabre Medicament S.A.), BBI-801 (Sumitomo Dainippon Pharma Co., Ltd.), BION-004 (Aduro Biotech), CA-327 (Aurigene Discovery Technologies), CK-301 (Fortress Biotech, Inc.), ENUM 244C8 (Enumeral Biomedical Holdings, Inc.), FPT155 (Five Prime Therapeutics, Inc.), FS118 (F-star Alpha Ltd.), hAb21 (Stainwei Biotech, Inc.), J43 (Transgene S.A.), JTX-4014 (Jounce Therapeutics, Inc.), KD033 (Kadmon Holdings, Inc.), KY-1003 (Kymab Ltd.), MCLA-134 (Merus B.V.), MCLA-145 (Merus B.V.), PRS-332 (Pieris AG), SHR-1316 (Atridia Pty Ltd.), STI-A1010 (Sorrento Therapeutics, Inc.), STI-A1014 (Sorrento Therapeutics, Inc.), STI-A1110 (Les Laboratoires Servier), and XmAb20717 (Xencor, Inc.).

The second therapeutic agent for use in the methods described in the present invention may be directed or targeted to, for example, 5T4; A33; alpha-folate receptor 1 (e.g. mirvetuximab soravtansine); Alk-1; BCMA (e.g. see WO2016166629 and others disclosed herein); BTN1A1 (e.g. see WO2018222689); CA19-9; CA-125 (e.g. abagovomab); Carboanhydrase IX; CCR2; CCR4 (e.g. mogamulizumab); CCR5 (e.g. leronlimab); CCR8; CD3 [e.g. blinatumomab (CD3/CD19 bispecific), PF-06671008 (CD3/P-cadherin bispecific), PF-06863135 (CD3/BCMA bispecific)]; CD19 (e.g. blinatumomab, MOR208); CD20 (e.g. ibritumomab tiuxetan, obinutuzumab, ofatumumab, rituximab, ublituximab); CD22 (inotuzumab ozogamicin, moxetumomab pasudotox); CD25; CD28; CD30 (e.g. brentuximab vedotin); CD33 (e.g. gemtuzumab ozogamicin); CD38 (e.g. daratumumab, daratumumab and hyaluronidase, and isatuximab), CD40; CD-40L; CD44v6; CD47 (e.g. Hu5F9-G4, CC-90002, SRF231, B6H12); CD52 (e.g. alemtuzumab); CD56; CD63; CD79 (e.g. polatuzumab vedotin); CD80; CD86; CD123; CD276 / B7-H3 (e.g. omburtamab); CDH17; CEA; ClhCG; CTLA-4 (e.g. ipilimumab, tremelimumab), CXCR4; desmoglein 4; DLL3 (e.g. rovalpituzumab tesirine); DLL4; E-cadherin; EDA; EDB; EFNA4; EGFR (e.g. cetuximab, depatuxizumab mafodotin, necitumumab, panitumumab); EGFRvlll; Endosialin; EpCAM (e.g. oportuzumab monatox); FAP; Fetal Acetylcholine Receptor; FLT3 (e.g. see WO2018/220584); 4-1 BB (CD137) [e.g. utomilumab/PF-05082566 (see WO2012/032433) or urelumab/BMS-663513], GD2 (e.g. dinutuximab, 3F8); GD3; GITR (e.g. TRX518); GloboH; GM1; GM2; HER2/neu [e.g. margetuximab, pertuzumab, trastuzumab; ado-trastuzumab emtansine, trastuzumab duocarmazine, PF-06804103 (see US8828401)]; HER3; HER4; ICOS; IL-10; ITG-AvB6; LAG-3 (e.g. relatlimab, IMP701); Lewis-Y; LG; Ly-6; M-CSF [e.g. PD-0360324 (see US7326414)]; (membrane-bound) IgE; MCSP; mesothelin; MIS Receptor type II; MUC1; MUC2; MUC3; MUC4; MUC5AC; MUC5B; MUC7; MUC16; Notch1; Notch3; Nectin-4 (e.g. enfortumab vedotin); OX40 [e.g. PF-04518600 (see US7960515)]; P-Cadherin [e.g. PF-06671008 (see WO2016/001810)]; PCDHB2; PD-1 [e.g. BCD-100, camrelizumab, cemiplimab, genolimzumab (CBT-501), MEDI0680, nivolumab, pembrolizumab, sasanlimab (PF-06801591, see WO2016/092419), sintilimab, spartalizumab, STI-A1110, tislelizumab, TSR-042, and others disclosed herein]; PD-L1 (e.g. atezolizumab, durvalumab, BMS-936559 (MDX-1105), LY3300054, and others disclosed herein); PDGFRA (e.g. olaratumab); Plasma Cell Antigen; PolySA; PSCA; PSMA; PTK7 [e.g. PF-06647020 (see US9409995)]; Ror1; SAS; SLAMF7 (e.g. elotuzumab); SHH; SIRPa (e.g. ED9, Effi-DEM); STEAP; sTn; TGF-beta; TIGIT; TIM-3; TMPRSS3; TNF-alpha precursor; TROP-2 (e.g., sacituzumab govitecan); TSPAN8; VEGF (e.g. bevacizumab, brolucizumab); VEGFR1 (e.g. ranibizumab); VEGFR2 (e.g. ramucirumab, ranibizumab); and Wue-1.

### Treatment

Each therapeutic agent in the treatment methods described herein (e.g., Compound 1 and one or more additional therapeutic agents), can be administered either alone or in a medicament (also referred to herein as a pharmaceutical composition) which comprises the therapeutic agent and one or more pharmaceutically acceptable carriers, excipients and diluents, according to standard pharmaceutical practice.

Each therapeutic agent in the methods described herein can be administered, either alone as a monotherapy, or in combination with one or more additional therapeutic agents, by any suitable enteral route or parenteral route of administration. The term "enteral route" of administration refers to the administration via any part of the gastrointestinal tract. Examples of enteral routes include oral, mucosal, buccal, and rectal route, or intragastric route. "Parenteral route" of administration refers to a route of administration other than enteral route. Examples of parenteral routes of administration include intravenous, intramuscular, intradermal, intraperitoneal, intratumor, intravesical, intraarterial, intrathecal, intracapsular, intraorbital, intracardiac, transtracheal, intraarticular, subcapsular, subarachnoid, intraspinal, epidural and intrasternal, subcutaneous, or topical administration. The therapeutic agents of the disclosure can be administered using any suitable method, such as by oral ingestion, nasogastric tube, gastrostomy tube, injection, infusion, implantable infusion pump, and osmotic pump. The suitable route and method of administration may vary depending on a number of factors such as the specific therapeutic agent being used, the rate of absorption desired, specific formulation or dosage form used, type or severity of the disorder being treated, the specific site of action, and conditions of the patient.

Oral administration of a solid dose form of a therapeutic agent may be, for example, presented in discrete units, such as hard or soft capsules, pills, cachets, lozenges, or tablets, each containing a predetermined amount of at least one therapeutic agent. In another aspect, the oral administration may be in a powder or granule form. In another aspect, the oral dose form is sub-lingual, such as, for example, a lozenge. In such solid dosage forms, therapeutic agents are ordinarily combined with one or more adjuvants. Such capsules or tablets may contain a controlled-release formulation. In the case of capsules, tablets, and pills, the dosage forms also may comprise buffering agents or may be prepared with enteric coatings.

In another aspect, oral administration of a therapeutic agent may be in a liquid dose form. Liquid dosage forms for oral administration include, for example, pharmaceutically acceptable emulsions, solutions, suspensions, syrups, and elixirs containing inert diluents commonly used in the art (e.g., water). Such compositions also may comprise adjuvants, such as wetting, emulsifying, suspending, flavoring (e.g., sweetening), and/or perfuming agents.

Accordingly, for example, in some embodiments, Compound 1, or a pharmaceutically acceptable salt thereof, is administered as a monotherapy in a dosage form selected from one or more tablets, one or more capsules, a liquid solution, a liquid suspension or a syrup. In some embodiments the dosage form is one or more tablets, preferably a single tablet.

In some aspects, therapeutic agents are administered in a parenteral dose form. "Parenteral administration" includes, for example, subcutaneous injections, intravenous injections, intraperitoneal injections, intramuscular injections, intrasternal injections, and infusion. Injectable preparations (i.e., sterile injectable aqueous or oleaginous suspensions) may be formulated according to the known art using suitable dispersing, wetting, and/or suspending agents, and include depot formulations.

For example, in some embodiments, Compound 1, or a pharmaceutically acceptable salt thereof, is administered in a dosage form selected from one or more tablets, one or more capsules, a liquid solution, a liquid suspension or a syrup, and a second therapeutic agent (e.g., a chemotherapeutic agent such as docetaxel or a biotherapeutic agent such as a PD-1 or PD-L1 antibody) is administered intravenously.

In some aspects, therapeutic agents are administered in a topical dose form. "Topical administration" includes, for example, transdermal administration, such as via transdermal patches or iontophoresis devices, intraocular administration, or intranasal or inhalation administration. Compositions for topical administration also include, for example, topical gels, sprays, ointments, and creams. A topical formulation may include a compound that enhances absorption or penetration of the active ingredient through the skin or other affected areas. When therapeutic agents are administered by a transdermal device, administration will be accomplished using a patch either of the reservoir and porous membrane type or of a solid matrix variety. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated--see, for example, Finnin and Morgan, J. Pharm. Sci., 88 (10), 955-958 (1999).

Other carrier materials and modes of administration known in the pharmaceutical art may also be used with therapeutic agents. The above considerations in regard to effective formulations and administration procedures are well known in the art and are described in standard textbooks. Formulation of drugs is discussed in, for example, Hoover, John E., Remington's Pharmaceutical Sciences, Mack Publishing Co., Easton, Pa., 1975; Liberman et al., Eds., Pharmaceutical Dosage Forms, Marcel Decker, New York, N.Y., 1980; and Kibbe et al., Eds., Handbook of Pharmaceutical Excipients (3.sup.rd Ed.), American Pharmaceutical Association, Washington, 1999.

For the combination treatment, selecting a dosage regimen (also referred to herein as an administration regimen) for the methods described herein may depend on several factors, including the serum or tissue turnover rate of the entity, the level of symptoms, the immunogenicity of the entity, and the accessibility of the target cells, tissue or organ in the subject being treated. Preferably, a dosage regimen maximizes the amount of each therapeutic agent delivered to the patient consistent with an acceptable level of side effects. Accordingly, the dose amount and dosing frequency of each therapeutic agent or chemotherapeutic agent in the combination depends in part on the particular therapeutic agent, the severity of the cancer being treated, and patient characteristics. Guidance in selecting appropriate doses of antibodies, cytokines, and small molecules are available. See, e.g., Wawrzynczak (1996) Antibody Therapy, Bios Scientific Pub. Ltd, Oxfordshire, UK; Kresina (ed.) (1991) Monoclonal Antibodies, Cytokines and Arthritis, Marcel Dekker, New York, NY; Bach (ed.) (1993) Monoclonal Antibodies and Peptide Therapy in Autoimmune Diseases, Marcel Dekker, New York, NY; Baert et al. (2003) New Engl. J. Med. 348:601-608; Milgrom etal. (1999) New Engl. J. Med. 341:1966-1973; Slamon et al. (2001) New Engl. J. Med. 344:783-792; Beniaminovitz et al. (2000) New Engl. J. Med. 342:613-619; Ghosh et al. (2003) New Engl. J. Med. 348:24-32; Lipsky et al. (2000) New Engl. J. Med. 343:1594-1602; Physicians' Desk Reference 2003 (Physicians' Desk Reference, 57th Ed); Medical Economics Company; ISBN: 1563634457; 57th edition (November 2002). Determination of the appropriate dosage regimen may be made by the clinician, e.g., using parameters or factors known or suspected in the art to affect treatment or predicted to affect treatment, and will depend, for example, the patient's clinical history (e.g., previous therapy), the type and stage of the cancer to be treated and biomarkers of response to one or more of the therapeutic agents in the combination therapy.

In some aspects, a subject can be administered with a fixed dose of each therapeutic agent (e.g., Compound 1 in monotherapy or in combination therapy with one or more additional therapeutic agents such as chemotherapeutic agents or biotherapeutic agents) of about or of at least about 0.05 µg, 0.2 µg, 0.5 µg, 1 µg, 10 µg, 100 µg, 0.1 mg, 0.2 mg, 0.3 mg, 0.4 mg, 0.5 mg, 0.6 mg, 0.7 mg, 0.8 mg, 0.9 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 75 mg, 80 mg, 90 mg, 100 mg, 125 mg, 150 mg, 175 mg, 200 mg, 225 mg, 250 mg, 275 mg, 300 mg, 350 mg, 400 mg, 450 mg, 500 mg, 550 mg, 600 mg, 350 mg, 700 mg, 750 mg, 800 mg, 900 mg, 1000 mg or 1500 mg, or higher. The fixed dose may be administered at intervals of, e.g. daily, every other day, three times per week, or one time each week, two weeks, three weeks, monthly, once every 2 months, once every 3 months, once every 4 months, etc.

In some aspects, each therapeutic agent (e.g., Compound 1 in monotherapy or in combination therapy with one or more additional therapeutic agents such as chemotherapeutic agents or biotherapeutic agents) of the treatment methods described herein can be administered to a subject at a dose from about 0.05 µg/kg to about 1000 mg/kg, from about 2 mg/kg to about 900 mg/kg, from about 3 mg/kg to about 800 mg/kg, from about 4 mg/kg to about 700 mg/kg, from about 5 mg/kg to about 600 mg/kg, from about 6 mg/kg to about 550 mg/kg, from about 7 mg/kg to about 500 mg/kg, from about 8 mg/kg to about 450 mg/kg, from about 9 mg/kg to about 400 mg/kg, from about 5 mg/kg to about 200 mg/kg, from about 2 mg/kg to about 150 mg/kg, from about 5 mg/kg to about 100 mg/kg, from about 10 mg/kg to about 100 mg/kg, or from about 10 mg/kg to about 60 mg/kg. For example, the second therapeutic agent can be administered to a subject at a dose of at least about 0.05 µg/kg, 0.2 µg/kg, 0.5 µg/kg, 1 µg/kg, 10 µg/kg, 100 µg/kg, 0.2 mg/kg, 1.0 mg/kg, 2.0 mg/kg, 3.0 mg/kg, 5.0 mg/kg, 10 mg/kg, 25 mg/kg, 50 mg/kg body weight or more. See, e.g., Yang et al. (2003) New Engl. J. Med. 349:427-434; Herold et al. (2002) New Engl. J. Med. 346:1692-1698; Liu et al. (1999) J. Neurol. Neurosurg. Psych. 67:451-456; Portielji et al. (20003) Cancer Immunol. Immunother. 52:133-144.

In some aspects, each therapeutic agent (e.g., Compound 1 in monotherapy or in combination therapy with one or more additional therapeutic agents such as chemotherapeutic agents or biotherapeutic agents) of the treatment methods described herein can be administered to a subject at a dose from about 1 mg/m² to about 3000 mg/m², from about 2 mg/m² to about 2000 mg/m², from about 3 mg/m² to about 1000 mg/m², from about 4 mg/m² to about 750 mg/m², from about 5 mg/m² to about 600 mg/m², from about 6 mg/m² to about 550 mg/m², from about 7 mg/m² to about 500 mg/m², from about 8 mg/m² to about 450 mg/m², from about 9 mg/m² to about mg/m². For example, the second therapeutic agent can be administered to a subject at a dose of at least about 5 mg/m², 10 mg/m², 15 mg/m², 20 mg/m², 25 mg/m², 30 mg/m², 35 mg/m², 40 mg/m², 45 mg/m², 50 mg/m², 55 mg/m², 60 mg/m², 65 mg/m², 70 mg/m², 75 mg/m², 80 mg/m², 85 mg/m², 90 mg/m², 95 mg/m², 100 mg/m², 105 mg/m², 110 mg/m², 115 mg/m², 120 mg/m², 130 mg/m², 135 mg/m², 140 mg/m², 145 mg/m², 150 mg/m², 155 mg/m², 160 mg/m², 165 mg/m², 170 mg/m², 175 mg/m², 180 mg/m², 185 mg/m², 190 mg/m², 195 mg/m², or 200 mg/m².

In some aspects, the second therapeutic agents described herein may be administered at least once a day, twice a day, three times a day, four times a day, once every two days, once every three days, once a week, once every two weeks, once every three weeks, once every four weeks, once every 30 days, once every five weeks, once every six weeks, once a month, once every two months, once every three months, or once every four months in an oral, IV (intravenous) or SC (subcutaneous) dose.

The treatment methods described herein can continue for as long as the clinician overseeing the patient's care deems the treatment method to be effective. Non-limiting parameters that indicate the treatment method is effective include any one or more of the following: tumor shrinkage (in terms of weight and/or volume); a decrease in the number of individual tumor colonies; tumor elimination; and progression-free survival. Change in tumor size may be determined by any suitable method such as imaging. Various diagnostic imaging modalities well known in the art can be employed, such as computed tomography (CT scan), dual energy CDT, positron emission tomography, ultrasound, CAT scan and MRI. In some aspects, a combination therapy of the invention is used to treat a tumor that is large enough to be found by palpation or by imaging techniques well known in the art, such as MRI, ultrasound, or CAT scan.

Exemplary lengths of time associated with the course of therapy include about one week; about two weeks; about three weeks; about four weeks; about five weeks; about six weeks; about seven weeks; about eight weeks; about nine weeks; about ten weeks; about eleven weeks; about twelve weeks; about thirteen weeks; about fourteen weeks; about fifteen weeks; about sixteen weeks; about seventeen weeks; about eighteen weeks; about nineteen weeks; about twenty weeks; about twenty-one weeks; about twenty-two weeks; about twenty-three weeks; about twenty four weeks; about seven months; about eight months; about nine months; about ten months; about eleven months; about twelve months; about thirteen months; about fourteen months; about fifteen months; about sixteen months; about seventeen months; about eighteen months; about nineteen months; about twenty months; about twenty one months; about twenty-two months; about twenty-three months; about twenty-four months; about thirty months; about three years; about four years, and about five years.

Accordingly, in some embodiments, Compound 1, or a pharmaceutically acceptable salt thereof, is administered for seven consecutive days, either alone as a monotherapy or in combination with one or more additional therapeutic agents (e.g., chemotherapeutic agents or biotherapeutic agents).

In some embodiments, the therapeutic agents described herein can be administered for one cycle comprising seven consecutive days, fourteen consecutive days, twenty-one consecutive days, twenty-eight consecutive days, thirty-five consecutive days, forty-two consecutive days, forty-nine consecutive days, fifty-six consecutive days, sixty-three consecutive days, or seventy consecutive days. In some embodiments, the administration takes place for at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more cycles. In some embodiments, each new cycle begins the day after the end of the previous cycle. In other embodiments, each new cycle begins 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 1 month, 2 months, 3 months, or 4 months after the previous cycle.

In some embodiments, each therapeutic agent can have different days in one cycle. For example, Compound 1, or a pharmaceutically acceptable salt thereof, is administered for one cycle comprising twenty-eight days, and a second therapeutic agent such as docetaxel is administered for one cycle comprising twenty-one days. In some of these embodiments, administration takes place for 1, 2, 3, 4, 5, 6, 7, 8, 9,10, 11, 12, 13, 14, 15, 16, 17, 18, 19, 20, 21, 22, 23, 24, 25, 26, 27, or 28 cycles. In some embodiments, administration takes place for 4 cycles, for 6 cycles, for 12 cycles, or for 24 cycles.

In some embodiments, Compound 1, or a pharmaceutically acceptable salt thereof, is administered for one cycle comprising twenty-eight consecutive days as a monotherapy. In some of these embodiments, administration takes place for 1, 2, 3, 4, 5, 6, 7, 8, 9,10, 11, 12, 13, 14, 15, 16, 17, 18, 19, 20, 21, 22, 23, 24, 25, 26, 27, or 28 cycles. In some embodiments, administration takes place for 4 cycles, for 6 cycles, for 12 cycles, or for 24 cycles. In some embodiments, each new cycle begins the day after the end of the previous cycle. In other embodiments, each new cycle begins 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 1 month, 2 months, 3 months, or 4 months after the previous cycle.

In certain embodiments of the invention, the patient is administered 1 mg, 2 mg, 4 mg, 6 mg, 8 mg, 12 mg, 16 mg, 32 mg, 60 mg, 120 mg or more of Compound 1 QD (once daily).

In some embodiments, administration of Compound 1 takes place while the patient is fasted. In other embodiments, administration of Compound 1 takes place while the patient is fed.

In certain embodiments of the invention, the patient is administered 1 mg of Compound 1 QD (once daily). In certain embodiments of the invention, the patient is administered 2 mg of Compound 1 QD (once daily). In certain embodiments of the invention, the patient is administered 4 mg of Compound 1 QD (once daily). In certain embodiments of the invention, the patient is administered 6 mg of Compound 1 QD (once daily). In certain embodiments of the invention, the patient is administered 8 mg of Compound 1 QD (once daily). In certain embodiments of the invention, the patient is administered 12 mg of Compound 1 QD (once daily). In certain embodiments of the invention, the patient is administered 16 mg of Compound 1 QD (once daily). In certain embodiments of the invention, the patient is administered 30 mg of Compound 1 QD (once daily). In certain embodiments of the invention, the patient is administered 32 mg of Compound 1 QD (once daily). In certain embodiments of the invention, the patient is administered 60 mg of Compound 1 QD (once daily). In certain embodiments of the invention, the patient is administered 120 mg of Compound 1 QD (once daily).

In some embodiments, the patient is administered with 1 mg, 2 mg, 4 mg, 6 mg, 8 mg, 12 mg, 16 mg, 30 mg, 32 mg, 60 mg, or 120 mg of Compound 1 QD (once daily), and 60 mg/m², 65 mg/m², 70 mg/m², 75 mg/m², 80 mg/m², 85 mg/m², 90 mg/m², 95 mg/m², or 100 mg/m² of a second therapeutic agent such as docetaxel once every 21 days (e.g., administering over 1 hour on day 1 of a cycle comprising 21 days). In some embodiments, Compound 1 is administered for one cycle comprising twenty-eight days, and the second therapeutic agent such as docetaxel is administered for one cycle comprising twenty-one days. In some embodiments, administration takes place for 1, 2, 3, 4, 5, 6, 7, 8, 9,10, 11, 12, 13, 14, 15, 16, 17, 18, 19, 20, 21, 22, 23, 24, 25, 26, 27, or 28 cycles. In some embodiments, administration takes place for 4 cycles, for 6 cycles, for 12 cycles, or for 24 cycles.

In some embodiments, the patient is administered with 1 mg, 2 mg, 4 mg, 6 mg, 8 mg, 12 mg, 16 mg, 30 mg, 32 mg, 60 mg, or 120 mg of Compound 1 QD (once daily) for one cycle comprising twenty-eight days, and 60 mg/m², 65 mg/m², 70 mg/m², 75 mg/m², 80 mg/m², 85 mg/m², 90 mg/m², 95 mg/m², or 100 mg/m² of a second therapeutic agent such as docetaxel once every 21 days (e.g., administering over 1 hour on day 1 of a cycle comprising 21 days).

In some embodiments, the patient is administered with 1 mg, 2 mg, 4 mg, 6 mg, 8 mg, 12 mg, 16 mg, 30 mg, 32 mg, 60 mg, or 120 mg of Compound 1 QD (once daily) for one cycle comprising twenty-eight days, and 60 mg/m², 65 mg/m², 70 mg/m², 75 mg/m², 80 mg/m², 85 mg/m², 90 mg/m², 95 mg/m², or 100 mg/m² of a second therapeutic agent such as docetaxel once every 21 days (e.g., administering over 1 hour on day 1 of a cycle comprising 21 days), and the administration takes place for 4 cycles, for 6 cycles, for 12 cycles, or for 24 cycles.

In some embodiments, the patient is administered with 1 mg, 2 mg, 4 mg, 6 mg, 8 mg, 12 mg, 16 mg, 30 mg, 32 mg, 60 mg, or 120 mg of Compound 1 QD (once daily), and a second therapeutic agent such as a PD-1 or a PD-L1 antibody.

In some aspects, the first therapeutic agent (i.e., Compound 1) may be administered simultaneously (i.e., in the same medicament), concurrently (i.e., in separate medicaments administered one right after the other in any order) or sequentially in any order with the second (or more) therapeutic agent. Sequential administration is particularly useful when the therapeutic agents are in different dosage forms (e.g., Compound 1 is a tablet or capsule and the second therapeutic agent is a sterile liquid) and/or are administered on different dosing schedules. For example, Compound 1 is administered once daily and the second therapeutic agent is administered less frequently, such as once weekly, once every two weeks, or once every three weeks.

In some aspects, the second therapeutic agent in the methods described herein can be administered using the same dosage regimen (dose, frequency and duration of treatment) that is typically employed when the agent is used as monotherapy for treating the same cancer. In other aspects, the subject may receive a lower total amount of at least one of the therapeutic agents than when the agent is used as monotherapy, e.g., smaller doses, less frequent doses, and/or shorter treatment duration.

In some embodiments, in a patient with locally advanced or metastatic breast cancer (e.g., after chemotherapy failure), such patient is administered with 1 mg, 2 mg, 4 mg, 6 mg, 8 mg, 12 mg, 16 mg, 30 mg, 32 mg, 60 mg, or 120 mg of Compound 1 QD orally, and with 60 mg/m² to 100 mg/m² of docetaxel once every 21 days intravenously (e.g., administering over 1 hour on day 1 of a cycle comprising 21 days). In some embodiments, Compound 1 is administered for one cycle comprising twenty-eight days, and the second therapeutic agent such as docetaxel is administered for one cycle comprising twenty-one days. In some embodiments, administration takes place for 1, 2, 3, 4, 5, 6, 7, 8, 9,10, 11, 12, 13, 14, 15, 16, 17, 18, 19, 20, 21, 22, 23, 24, 25, 26, 27, or 28 cycles. In some embodiments, administration takes place for 4 cycles, for 6 cycles, for 12 cycles, or for 24 cycles for Compound 1 and docetaxel.

In some embodiments, in a patient with locally advanced or metastatic breast cancer (e.g., after chemotherapy failure), such patient is administered with 1 mg, 2 mg, 4 mg, 6 mg, 8 mg, 12 mg, 16 mg, 30 mg, 32 mg, 60 mg, or 120 mg of Compound 1 QD orally for one cycle comprising twenty-eight days, and with 60 mg/m² to 100 mg/m² of docetaxel once every 21 days intravenously (e.g., administering over 1 hour on day 1 of a cycle comprising 21 days), and the administration takes place for 4 cycles, for 6 cycles, for 12 cycles, or for 24 cycles for Compound 1 and docetaxel.

In some embodiments, in a patient with breast cancer (e.g., operable node-positive breast cancer), such patient is administered with 1 mg, 2 mg, 4 mg, 6 mg, 8 mg, 12 mg, 16 mg, 30 mg, 32 mg, 60 mg, or 120 mg of Compound 1 QD orally, and with 75 mg/m² of docetaxel intravenously once every 21 days (e.g., administering over 1 hour on day 1 of a cycle comprising 21 days) after doxorubicin at 50 mg/m² and cyclophosphamide at 500 mg/m². In some embodiments, Compound 1 is administered for one cycle comprising twenty-eight days, and the second therapeutic agent such as docetaxel is administered for one cycle comprising twenty-one days. In some embodiments, administration takes place for 1, 2, 3, 4, 5, 6, 7, 8, 9,10, 11, 12, 13, 14, 15, 16, 17, 18, 19, 20, 21, 22, 23, 24, 25, 26, 27, or 28 cycles. In some embodiments, administration takes place for 4 cycles, for 6 cycles, for 12 cycles, or for 24 cycles for Compound 1, and for 6 cycles for docetaxel, doxorubicin, and cyclophosphamide (e.g., 21 days per cycle).

In some embodiments, in a patient with breast cancer (e.g., operable node-positive breast cancer), such patient is administered with 1 mg, 2 mg, 4 mg, 6 mg, 8 mg, 12 mg, 16 mg, 30 mg, 32 mg, 60 mg, or 120 mg of Compound 1 QD orally for one cycle comprising twenty-eight days, and with 75 mg/m² of docetaxel intravenously once every 21 days (e.g., administering over 1 hour on day 1 of a cycle comprising 21 days) after doxorubicin at 50 mg/m² and cyclophosphamide at 500 mg/m², and the administration takes place for 4 cycles, for 6 cycles, for 12 cycles, or for 24 cycles for Compound 1, and for 6 cycles for docetaxel, doxorubicin, and cyclophosphamide (e.g., 21 days per cycle).

In some embodiments, in a patient with locally advanced or metastatic NSCLC (e.g., after platinum therapy failure), such patient is administered with 1 mg, 2 mg, 4 mg, 6 mg, 8 mg, 12 mg, 16 mg, 30 mg, 32 mg, 60 mg, or 120 mg of Compound 1 QD orally, and with 75 mg/m² of docetaxel intravenously once every 21 days (e.g., administering over 1 hour on day 1 of a cycle comprising 21 days). In some embodiments, Compound 1 is administered for one cycle comprising twenty-eight days, and the second therapeutic agent such as docetaxel is administered for one cycle comprising twenty-one days. In some embodiments, administration takes place for 1, 2, 3, 4, 5, 6, 7, 8, 9,10, 11, 12, 13, 14, 15, 16, 17, 18, 19, 20, 21, 22, 23, 24, 25, 26, 27, or 28 cycles. In some embodiments, administration takes place for 4 cycles, for 6 cycles, for 12 cycles, or for 24 cycles for both Compound 1 and docetaxel.

In some embodiments, in a patient with locally advanced or metastatic NSCLC (e.g., after platinum therapy failure), such patient is administered with 1 mg, 2 mg, 4 mg, 6 mg, 8 mg, 12 mg, 16 mg, 30 mg, 32 mg, 60 mg, or 120 mg of Compound 1 QD orally for one cycle comprising twenty-eight days, and with 75 mg/m² of docetaxel intravenously once every 21 days (e.g., administering over 1 hour on day 1 of a cycle comprising 21 days), and the administration takes place for 4 cycles, for 6 cycles, for 12 cycles, or for 24 cycles for both Compound 1 and docetaxel.

In some embodiments, in a patient with unresectable, locally advanced or metastatic untreated NSCLC (e.g., chemotherapy-naive), such patient is administered with 1 mg, 2 mg, 4 mg, 6 mg, 8 mg, 12 mg, 16 mg, 30 mg, 32 mg, 60 mg, or 120 mg of Compound 1 QD orally, and with 75 mg/m² of docetaxel intravenously once every 21 days (e.g., administering over 1 hour on day 1 of a cycle comprising 21 days), and further followed by cisplatin 75 mg/m². In some embodiments, Compound 1 is administered for one cycle comprising twenty-eight days, and the second therapeutic agent such as docetaxel is administered for one cycle comprising twenty-one days. In some embodiments, administration takes place for 1, 2, 3, 4, 5, 6, 7, 8, 9,10, 11, 12, 13, 14, 15, 16, 17, 18, 19, 20, 21, 22, 23, 24, 25, 26, 27, or 28 cycles. In some embodiments, administration takes place for 4 cycles, for 6 cycles, for 12 cycles, or for 24 cycles for Compound 1 and docetaxel.

In some embodiments, in a patient with unresectable, locally advanced or metastatic untreated NSCLC (e.g., chemotherapy-naive), such patient is administered with 1 mg, 2 mg, 4 mg, 6 mg, 8 mg, 12 mg, 16 mg, 30 mg, 32 mg, 60 mg, or 120 mg of Compound 1 QD orally for one cycle comprising twenty-eight days, and with 75 mg/m² of docetaxel intravenously once every 21 days (e.g., administering over 1 hour on day 1 of a cycle comprising 21 days), and further followed by cisplatin 75 mg/m², and the administration takes place for 4 cycles, for 6 cycles, for 12 cycles, or for 24 cycles for Compound 1 and docetaxel.

In some embodiments, in a patient with squamous cell carcinoma of the head and neck cancer (SCCHN) (e.g., locally advanced), such patient is administered with 1 mg, 2 mg, 4 mg, 6 mg, 8 mg, 12 mg, 16 mg, 30 mg, 32 mg, 60 mg, or 120 mg of Compound 1 QD (once daily), and with 75 mg/m² of docetaxel intravenously once every 21 days (e.g., administering over 1 hour on day 1 of a cycle comprising 21 days), and further followed by cisplatin 75 mg/m² intravenously, followed by fluorouracil 750 mg/m² per day as a 24-hr IV on days 1-5, starting at end of cisplatin infusion. In some embodiments, Compound 1 is administered for one cycle comprising twenty-eight days, and the second therapeutic agent such as docetaxel is administered for one cycle comprising twenty-one days. In some embodiments, administration takes place for 1, 2, 3, 4, 5, 6, 7, 8, 9,10, 11, 12, 13, 14, 15, 16, 17, 18, 19, 20, 21, 22, 23, 24, 25, 26, 27, or 28 cycles. In some embodiments, administration takes place for 4 cycles, for 6 cycles, for 12 cycles, or for 24 cycles for Compound 1, and for 4 cycles for docetaxel, cisplatin, and fluorouracil (e.g., 21 days per cycle).

In some embodiments, in a patient with squamous cell carcinoma of the head and neck cancer (SCCHN) (e.g., locally advanced), such patient is administered with 1 mg, 2 mg, 4 mg, 6 mg, 8 mg, 12 mg, 16 mg, 30 mg, 32 mg, 60 mg, or 120 mg of Compound 1 QD (once daily) for one cycle comprising twenty-eight days, and with 75 mg/m² of docetaxel intravenously once every 21 days (e.g., administering over 1 hour on day 1 of a cycle comprising 21 days), and further followed by cisplatin 75 mg/m² intravenously, followed by fluorouracil 750 mg/m² per day as a 24-hr IV on days 1-5, starting at end of cisplatin infusion, and the administration takes place for 4 cycles, for 6 cycles, for 12 cycles, or for 24 cycles for Compound 1, and for 4 cycles for docetaxel, cisplatin, and fluorouracil (e.g., 21 days per cycle).

In some embodiments, in a patient with hormone refractory prostate cancer (HRPC) (e.g., androgen independent (hormone refractory) metastatic prostate cancer), such patient is administered with 1 mg, 2 mg, 4 mg, 6 mg, 8 mg, 12 mg, 16 mg, 30 mg, 32 mg, 60 mg, or 120 mg of Compound 1 QD orally, with 75 mg/m² of docetaxel intravenously once every 21 days (e.g., administering over 1 hour on day 1 of a cycle comprising 21 days), and with 5 mg prednisone twice a day continuously. In some embodiments, Compound 1 is administered for one cycle comprising twenty-eight days, and the second therapeutic agent such as docetaxel is administered for one cycle comprising twenty-one days. In some embodiments, administration takes place for 1, 2, 3, 4, 5, 6, 7, 8, 9,10, 11, 12, 13, 14, 15, 16, 17, 18, 19, 20, 21, 22, 23, 24, 25, 26, 27, or 28 cycles. In some embodiments, administration takes place for 4 cycles, for 6 cycles, for 12 cycles, or for 24 cycles for Compound 1, docetaxel, and prednisone.

In some embodiments, in a patient with hormone refractory prostate cancer (HRPC) (e.g., androgen independent (hormone refractory) metastatic prostate cancer), such patient is administered with 1 mg, 2 mg, 4 mg, 6 mg, 8 mg, 12 mg, 16 mg, 30 mg, 32 mg, 60 mg, or 120 mg of Compound 1 QD orally for one cycle comprising twenty-eight days, with 75 mg/m² of docetaxel intravenously once every 21 days (e.g., administering over 1 hour on day 1 of a cycle comprising 21 days), and with 5 mg prednisone twice a day continuously, and the administration takes place for 4 cycles, for 6 cycles, for 12 cycles, or for 24 cycles for Compound 1, docetaxel, and prednisone.

In some embodiments, in a patient with gastric adenocarcinoma (GC) (untreated, advanced GC, including the gastroesophageal junction), such patient is administered with 1 mg, 2 mg, 4 mg, 6 mg, 8 mg, 12 mg, 16 mg, 30 mg, 32 mg, 60 mg, or 120 mg of Compound 1 QD orally, with 75 mg/m² of docetaxel intravenously once every 21 days (e.g., administering over 1 hour on day 1 of a cycle comprising 21 days), followed by cisplatin 75 mg/m²once every 21 days (e.g., administering on day 1 of a cycle comprising 21 days), followed by fluorouracil 750 mg/m² per day as a 24-hour IV at days 1-5, starting at end of cisplatin infusion. In some embodiments, Compound 1 is administered for one cycle comprising twenty-eight days, and the second therapeutic agent such as docetaxel is administered for one cycle comprising twenty-one days. In some embodiments, administration takes place for 1, 2, 3, 4, 5, 6, 7, 8, 9,10, 11, 12, 13, 14, 15, 16, 17, 18, 19, 20, 21, 22, 23, 24, 25, 26, 27, or 28 cycles. In some embodiments, administration takes place for 4 cycles, for 6 cycles, for 12 cycles, or for 24 cycles for Compound 1, docetaxel, cisplatin, and fluorouracil.

In some embodiments, in a patient with gastric adenocarcinoma (GC) (untreated, advanced GC, including the gastroesophageal junction), such patient is administered with 1 mg, 2 mg, 4 mg, 6 mg, 8 mg, 12 mg, 16 mg, 30 mg, 32 mg, 60 mg, or 120 mg of Compound 1 QD orally for one cycle comprising twenty-eight days, with 75 mg/m² of docetaxel intravenously once every 21 days (e.g., administering over 1 hour on day 1 of a cycle comprising 21 days), followed by cisplatin 75 mg/m²once every 21 days (e.g., administering on day 1 of a cycle comprising 21 days), followed by fluorouracil 750 mg/m² per day as a 24-hour IV at days 1-5, starting at end of cisplatin infusion, and the administration takes place for 4 cycles, for 6 cycles, for 12 cycles, or for 24 cycles for Compound 1, docetaxel, cisplatin, and fluorouracil.

In some aspects, the treatment methods described herein may be used prior to or following surgery to remove a tumor and may be used prior to, during or after radiation therapy.

In some aspects, the treatment methods described herein is administered to a patient who has not been previously treated with a therapeutic or chemotherapeutic agent, i.e., is treatment-naïve. In other aspects, the combination therapy is administered to a patient who failed to achieve a sustained response after prior therapy with a therapeutic or chemotherapeutic agent, i.e., is treatment-experienced. In some aspects, the subject has received a prior therapy to treat the tumor and the tumor is relapsed, metastatic, or refractory.

Accordingly, for example, in some embodiments, the patients with NSCLC receiving the treatment methods described herein have progressed after at least first line of checkpoint inhibitors (such as PD-1 or PD-L1 inhibitors such as ipilimumab, nivolumab, pembrolizumab, atezolizumab, avelumab, durvalumab, or cemiplimab-rwlc) and first line of platinum based chemotherapy (second line + (2L+)). In some embodiments, the patients with urothelial carcinoma receiving the treatment methods described herein have progressed after at least first line of standard of care systemic chemotherapy (cisplatin/carboplatin + gemcitabine; or methotrexate/vinblastine sulfate/doxorubicin hydrochloride/cisplatin and checkpoint inhibitor (2L+). In some embodiments, the patients with HNSCC receiving the treatment methods described herein have progressed after at least first line of standard of care systemic chemotherapy and first line of checkpoint inhibitor (2L+).

In some embodiments, the patient is premedicated with other therapeutic agents (e.g., corticosteroids such as dexamethasone) prior to the treatment methods described herein. For example, the patient is premedicated with oral corticosteroids such as dexamethasone 16 mg per day (e.g., 8 mg BID) for 3 days starting 1 day prior to receiving treatment methods described herein (e.g., monotherapy or combination therapy).

Encompassed by the invention provided herein also include combination therapies that have additive potency or an additive therapeutic effect while reducing or avoiding unwanted or adverse effects. The invention also encompasses synergistic combinations where the therapeutic efficacy is greater than additive, while unwanted or adverse effects are reduced or avoided. In certain aspects, the methods and compositions provided herein permit treatment or prevention of diseases and disorders wherein treatment is improved by an enhanced anti-tumor response using lower and/or less frequent doses of at least therapeutic agent in a combination therapy to at least one of: i) reduce the incidence of unwanted or adverse effects caused by the administration of the therapeutic agents separately, while at least maintaining efficacy of treatment; ii) increase patient compliance, and iii) improve efficacy of the anti-tumor treatment.

### Kits

The therapeutic agents of the treatment methods described herein may conveniently be in the form of a kit for administration of the compositions.

In some embodiments, a kit comprises a container and a package insert. The container contains at least one dose of Compound 1, and the package insert / label comprises instructions for treating a patient for cancer and/or cancer-associated disease using Compound 1. The container may be comprised of a specific shape (e.g., vials, syringes and bottles) and/or material (e.g., plastic or glass). The kit may further comprise other materials that may be useful in administering Compound 1, such as diluents, filters, IV bags and lines, needles and syringes.

In some embodiments, a kit comprises at least a first container and a second container and a package insert. The first container contains at least one dose of Compound 1, and the second container contains at least one dose of a second therapeutic agent of the combination therapy. The package insert / label comprises instructions for treating a patient for cancer and/or cancer-associated disease using the therapeutic agents. The first and second containers may be comprised of the same or different shape (e.g., vials, syringes and bottles) and/or material (e.g., plastic or glass). The kit may further comprise other materials that may be useful in administering the therapeutic agents, such as diluents, filters, IV bags and lines, needles and syringes.

### EXAMPLES

Without further elaboration, it is believed that one skilled in the art can, using the preceding description, practice the present invention to its fullest extent. The following detailed examples describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

### COMPARATIVE EXAMPLE 1

### Determination of Day 1 and Day 15 PK profiles of Compound 1, BID Dosing

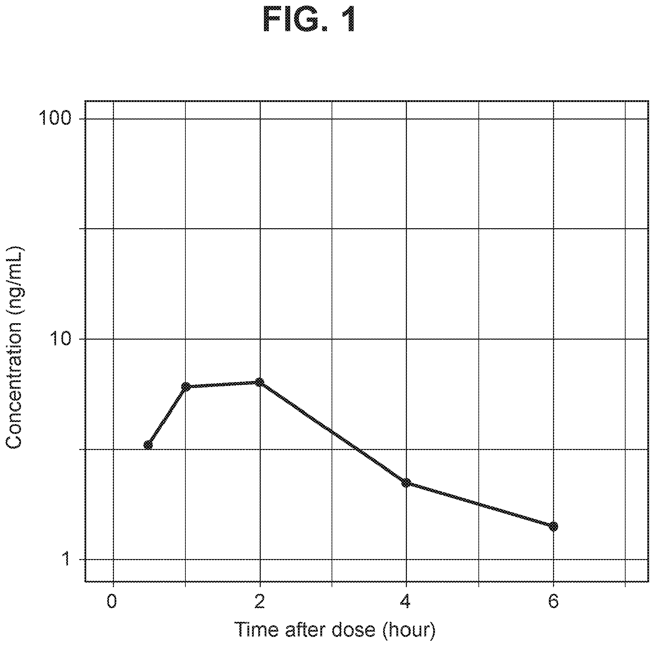

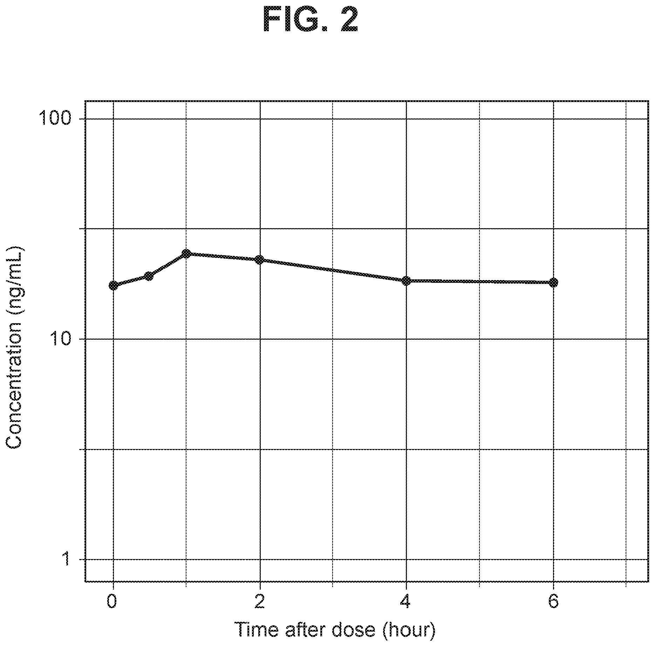

Blood samples for the characterization of PK profiles were collected at pre-and post-dose on Day 1 (single dose) and Day 15 (steady state), and were analyzed for Compound 1 using a validated liquid chromatography coupled with tandem mass spectrometry (LC-MS/MS) assay. The lower limit of quantitation of the assay was 0.250 ng/mL. The representative day 1 median PK profile of Compound 1 dosed at 2 mg BID is provided at Fig. 1. The representative steady state (day 15) median PK profile of Compound 1 dosed at 2 mg BID is provided at Fig. 2. Table 1 (below) depicts representative mean maximum (Cmax) and minimum (Cmin) daily concentrations of Compound 1 at "steady state" (day 15) dosed BID at 2 mg.

| **TABLE 1** | | | |
|---|---|---|---|
| Dose | N | Cₘₐₓ (ng/mL) | Cₘᵢₙ (ng/mL) |
| 2 mg BID | 2 | 26.9 | 14.6 |

### EXAMPLE 1

### Determination of Steady State Concentration Profiles, QD and BID

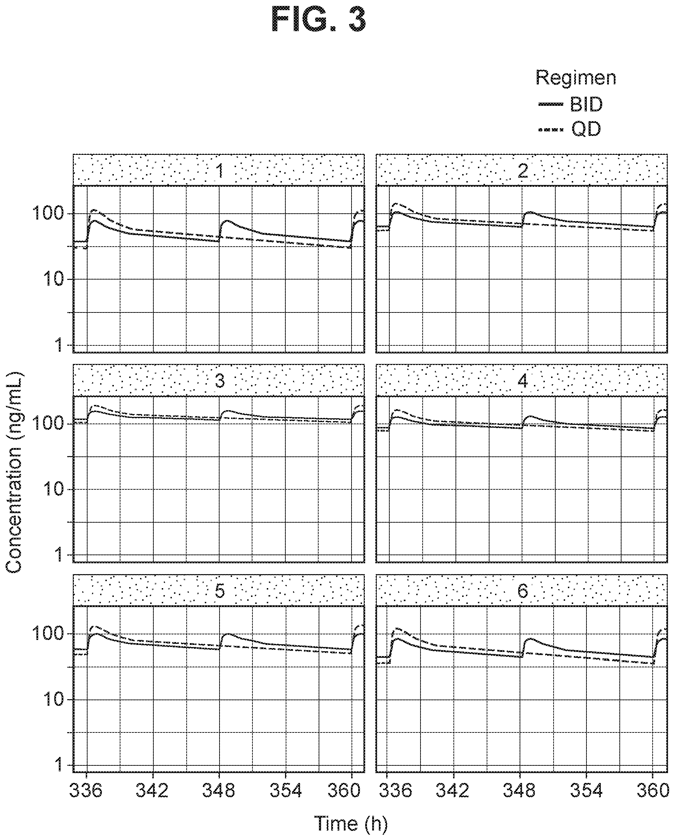

A population pharmacokinetic (PK) model was generated based on the preliminary PK data from 6 patients received Compound 1 orally at doses up to 2 mg BID at steady state. It was observed that actual mean peak to trough ratio at steady state (here, 15 days of dosing) of Compound 1 in the NCT03854227 clinical trial was ~2 for BID dosing, substantially lower than that previously predicted based on pre-clinical data (~4 for BID). Furthermore, based on this population PK model, the steady state PK profiles following QD vs BID dosing (using a 16 mg total daily dose as an example) were compared using the individual PK parameters from each of these 6 patients. The results are provided at FIG. 3, and show that QD dosing yields low peak-to-trough ratios (~4-fold or less) at steady state.

### EXAMPLE 2

### QD Dosing of Patient "A" with 32 mg Compound 1

Prior to administration and provision of Compound 1 to Patient "A" the investigator confirms appropriate temperature and storage conditions have been maintained. Records are checked to ensure Compound 1 has been stored in a secure, environmentally controlled, and monitored area with limited access. Patient "A" is instructed with respect to proper storage of Compound 1 to be administered at home.

Compound 1 is provided to Patient "A" as tablets (1 mg, 5 mg and 25 mg tablets supplied in separate labeled bottles) for oral administration. Because Patient "A" is receiving 32 mg QD he is instructed to swallow whole (i.e., without crushing or chewing) one 25 mg tablet, one 5 mg tablet, and two 1 mg tablets at the same time each morning with at least 8 oz (240 mL) of water on an empty stomach. No food or liquids other than water are be consumed for 2 hours before and 1 hour following each dose administration. Because Patient "A" visits the clinic weekly, he is provided by the clinic pharmacy with a one-week supply of Compound 1 at each visit. Patient "A" takes Compound 1 orally QD as described above at home for four cycles (a cycle is 28 days). Weekly visits to the clinic are made by the patient to evaluate the anti-cancer activity and side effects of Compound 1.

### EXAMPLE 3

### QD Dosing of Patient "B" with 60 mg Compound 1

Prior to administration and provision of Compound 1 to Patient "B" the investigator confirms appropriate temperature and storage conditions have been maintained. Records are checked to ensure Compound 1 has been stored in a secure, environmentally controlled, and monitored area with limited access. Patient "B" is instructed with respect to proper storage of Compound 1 to be administered at home.

Compound 1 is provided to Patient "B" as tablets (5 mg and 25 mg tablets supplied in separate labeled bottles) for oral administration. Because Patient "B" is receiving 60 mg QD she is instructed to swallow whole (i.e., without crushing or chewing) two 25 mg tablets and two 5 mg tablet at the same time each morning with at least 8 oz (240 mL) of water on an empty stomach. No food or liquids other than water are be consumed for 2 hours before and 1 hour following each dose administration. Because Patient "B" visits the clinic weekly, she is provided by the clinic pharmacy with a one-week supply of Compound 1 at each visit. Patient "B" takes Compound 1 orally QD as described above at home for twelve cycles (a cycle is 28 days). Weekly visits to the clinic are made by the patient to evaluate the anti-cancer activity and side effects of Compound 1.

### EXAMPLE 4

### QD Dosing of Patient "C" with 120 mg Compound 1

Prior to administration and provision of Compound 1 to Patient "C" the investigator confirms appropriate temperature and storage conditions have been maintained. Records are checked to ensure Compound 1 has been stored in a secure, environmentally controlled, and monitored area with limited access. Patient "C" is instructed with respect to proper storage of Compound 1 to be administered at home.

Compound 1 is provided to Patient "C" as tablets (5 mg and 25 mg tablets supplied in separate labeled bottles) for oral administration. Because Patient "C" is receiving 120 mg QD she is instructed to swallow whole (i.e., without crushing or chewing) four 25 mg tablets and four 5 mg tablet at the same time each morning with at least 8 oz (240 mL) of water on an empty stomach. No food or liquids other than water are be consumed for 2 hours before and 1 hour following each dose administration. Because Patient "C" visits the clinic weekly, she is provided by the clinic pharmacy with a one-week supply of Compound 1 at each visit. Patient "C" takes Compound 1 orally QD as described above at home for twenty-four cycles (a cycle is 28 days). Weekly visits to the clinic are made by the patient to evaluate the anti-cancer activity and side effects of Compound 1.

### EXAMPLE 5

### Phase 1, open-label, multi-center, dose escalation and dose expansion study to assess the safety, pharmacokinetics, pharmacodynamics, and anti-tumor activity of Compound 1 as a single agent and in combination in participants with locally advanced or metastatic selected solid tumor indications.

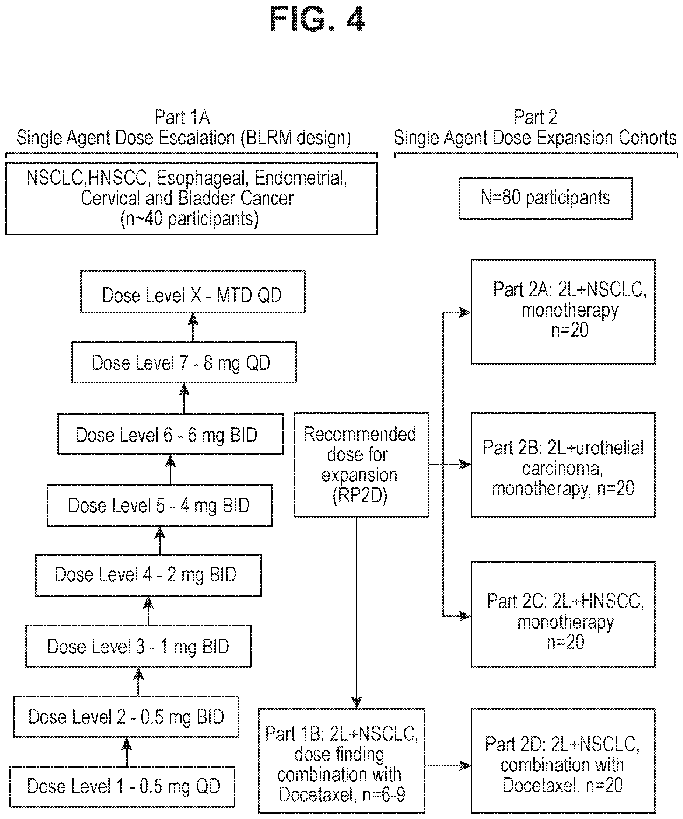

The study is divided into 2 parts, dose escalation (Part 1) followed by dose expansion (Part 2). The overall study design is depicted in FIG. 4. Part 1 dose escalation further divides into Part 1A and 1B. Part 1A contains dose escalation as a single agent in participants with locally advanced or metastatic head and neck squamous cell carcinoma (HNSCC), non-small cell lung cancer (NSCLC), esophageal cancer, endometrial cancer, cervical cancer, or bladder cancer who are resistant or intolerant to standard therapy or for whom no standard therapy is available, to determine the MTD (Maximum Tolerable Dose) and RP2D (Recommended Phase 2 Dose). Part 1B contains dose finding of Compound 1 in combination with docetaxel in locally advanced or metastatic NSCLC.

Part 1A (dose escalation) has approximately 9 cohorts and enrolls approximately 40 participants. A Bayesian Logistic Regression Model (BLRM) was used to determine the MTD. Participants received escalating doses of Compound 1 starting from 0.5 mg QD. Dose limiting toxicities (DLT) were assessed at the end of Cycle 1 (28 days) to inform dose escalation and determine the MTD. Cohort size was approximately 3 participants, with at least 1 DLT-evaluable participant per cohort at the first 3 dose levels and at least 2 DLT-evaluable participants per cohort in the remaining cohorts. Compound 1 was administered as a single agent, orally, once daily during the first cohort, twice a day (BID) or once a day (QD) for subsequent cohorts in 28 day cycles on a continuous basis until disease progression, participant refusal, or unacceptable toxicity. Other dosing regimens (e. g., a regimen with dose interruptions to allow recovery from certain adverse events) may be included in the study if further supported by emerging clinical data. The estimated length of treatment is approximately 2 years. Any additional treatment beyond 2 years is to be considered.

Part 2 (dose expansion) evaluates the safety and antitumor activity of Compound 1 monotherapy at the single agent RP2D in 3 expansion cohorts: locally advanced or metastatic NSCLC (Part 2A, 20 participants), urothelial carcinoma (Part 2B, 20 participants) and HNSCC (Part 2C, 20 participants). The collection of on-treatment biopsies in up to 5 participants per arm is strongly recommended if state, local, and institutional policies allow.

Furthermore, Compound 1 in combination with docetaxel is evaluated in locally advanced or metastatic NSCLC (Part 1B combination dose finding and Part 2D combination dose expansion). In part 1B, Compound 1 in combination with docetaxel is evaluated in participants with locally advanced or metastatic NSCLC who have progressed after at least 1 line of checkpoint inhibitor and platinum based chemotherapy (2L+). BLRM specifically designed for combinations guided by EWOC (Escalation With Overdose Control) principle is used for dose finding. Compound 1 starts at 1 dose level below single agent RP2D (RP2D-1) with fixed dose of docetaxel per standard of care. In addition, depending on the safety findings in Part 1A, and whether significant overlapping toxicities are expected in combination, the starting dose of Compound 1 can be further escalated or de-escalated but does not exceed the single agent RP2D. Participants are enrolled in cohorts of approximately 3 participants of whom at least 3 are DLT (Dose Limiting Toxicities) evaluable. Approximately 6-9 participants are enrolled in Part 1B.

Once the RP2D for the combination has been determined, additional NSCLC participants are enrolled under Part 2D combination expansion cohort until a total of 20 participants have been evaluated at the combination RP2D (Part 1B participants dosed at the combination RP2D are counted towards the total of 20 participants). The collection of on-treatment biopsies in up to 5 participants per arm is strongly recommended if state, local, and institutional policies allow.

Approximately 80 participants are enrolled in Part 2. The participant population is briefly described below. Monotherapy expansion cohorts Parts 2A, 2B, and 2C are listed below:
- Part 2A: Participants with NSCLC (n=20) who have progressed after at least 1 line of checkpoint inhibitors and 1 line of platinum based chemotherapy (2L+).
- Part 2B: Participants with urothelial carcinoma (n=20) who have progressed after at least 1 line of standard of care systemic chemotherapy (cisplatin/carboplatin+ Gemcitabine; or methotrexate/vinblastine sulfate/doxorubicin hydrochloride/cisplatin [MVAC]) and checkpoint inhibitor (2L+).
- Part 2C: Participants with HNSCC (n=20) who have progressed after at least 1 line of standard of care systemic chemotherapy and 1 line of checkpoint inhibitor (2L+).

Combination expansion cohort Part 2D (Compound 1 in combination with docetaxel) is listed below:
- Part 2D: Participants with NSCLC (n=20) who have progressed after at least 1 line of checkpoint inhibitor and 1 line of platinum based chemotherapy (2L+).
- All participants in Part 1 and Part 2:
   - Undergo up to 28 days of screening prior to study entry.
   - Receive doses of Compound 1, administered orally continuously on 28 day cycles up to 2 years (for combination with docetaxel, 21 day cycle is used to be consistent with docetaxel dosing schedule). Any additional treatment with Compound 1 beyond 2 years is to be discussed and approved by the Sponsor.
   - Treatment with Compound 1 continues until disease progression, a decision by the participant (withdrawal of consent or no longer willing to participate), or investigator, or unacceptable toxicity, whichever occurs first.
   - Undergo a Safety follow up visit at least 28 days but no more than 35 days after end of treatment.
   - In addition, participants in Part 1B and Part 2 dose expansion cohorts is contacted by telephone every 12 weeks for survival data collection until end of trial (2 years from last participant first dose).

A participant is considered to have completed the study if he/she has completed all phases of the study, including overall survival (OS). OS follow up does not continue after end of the study.

### Food-Effect Substudy

The effect of food on the PK of Compound 1 at the recommended Phase 2 dose and regimen (BID or QD) is assessed in at least 6 participants (but no more than 15 participants) from Part 2B, to inform whether Compound 1 can be administered without food restriction in subsequent clinical trials. A fixed sequence one-way cross-over design is utilized to assess the PK of Compound 1 under the fasted and fed conditions on Cycle 1 Day 15 and Day 16, respectively, following repeated dosing under empty stomach condition (no food or liquids other than water is consumed for 2 hours before and 1 hour following each dose).

With the exception of the study visits for food effect assessment (Cycle 1 Day 15 and Day 16), participants receive continuous oral doses of Compound 1 (QD or BID) under an empty stomach condition (i.e., no food or liquids other than water is consumed for 2 hours before and 1 hour following each dose). On Cycle 1 Day 15, participants receive an oral dose of Compound 1 under the fasted condition (overnight fasting of at least 10 hours; water permitted). On Cycle 1 Day 16, participants receive another oral dose of Compound 1 with a high-fat, high-calorie meal (breakfast) following 10-hour overnight fasting (water permitted). Serial PK samples are collected after each dose on Day 15 and Day 16. Subsequently, participants receive the remaining drug treatment under an empty stomach condition. Participants who have had a gastrectomy or have dietary or other restrictions that preclude a 10- hour overnight fast (water permitted) or consumption of the high fat, high calorie meal are not required to participate in this assessment.

### Inclusion Criteria

Participants are eligible to be included in the study only if all of the following criteria apply:
1. Females and/or male participants age ≥18 years.
2. For Part 1 dose escalation, participants must have histological or cytological diagnosis of a solid tumor that is advanced/metastatic, participants are intolerant to standard treatment or resistant to standard therapy* for the following tumor types:
   - NSCLC;
   - HNSCC;
   - Esophageal;
   - Endometrial;
   - Cervical;
   - Bladder.

   *Intolerance or progression on prior therapies must be documented for study enrollment.
3. For Part 2 single agent dose expansion, NSCLC participants:
   - Histologically or cytologically confirmed, locally advanced or metastatic NSCLC including squamous cell carcinoma or adenocarcinoma;
   - For NSCLC without available 1L targeted therapies (EGFR, ALK, ROS1, BRAF and NTRK negative), 2L+ NSCLC who have progressed after at least 1 line of checkpoint inhibitors (CPI) and 1 line of platinum based chemotherapy regimen given together or sequentially in the metastatic setting;
   - For NSCLC with available 1L targeted therapies (EGFR, ALK, ROS1, BRAF or NTRK positive), 2L+ NSCLC who have progressed after at least 1 line of targeted therapies;
   - Should receive no more than 3 lines of systemic therapies in metastatic setting;
   - Radiographic progression during or after the most recent treatment.
4. For Part 2 combination with docetaxel expansion, NSCLC participants:
   - Histologically or cytologically confirmed, locally advanced or metastatic NSCLC including both squamous cell carcinoma and adenocarcinoma;
   - For NSCLC without available 1L targeted therapies (EGFR, ALK, ROS1, BRAF and NTRK negative), 2L+ NSCLC who have progressed after at least 1 line of checkpoint inhibitors (CPI) and 1 line of platinum based chemotherapy regimen given together or sequentially in the metastatic setting;
   - For NSCLC with available 1L targeted therapies (EGFR, ALK, ROS1, BRAF or NTRK positive), 2L+ NSCLC who have progressed after at least 1 line of targeted therapies;
   - Have not been treated with taxane chemotherapy (docetaxel or paclitaxel) in locally advanced or metastatic setting;
   - Should receive no more than 2 lines of systemic therapies in metastatic setting;
   - Radiographic progression during or after the most recent treatment.
5. For Part 2 urothelial carcinoma participants:
   - Histologically or cytologically confirmed, locally advanced or metastatic urothelial carcinoma of the bladder, renal pelvis, ureter or urethra;
   - 2L+ urothelial carcinoma who have progressed after at least 1 line of standard of care chemotherapy (cisplatin/carboplatin+ Gemcitabine; or MVAC) and 1 line of checkpoint inhibitor in the metastatic setting;
   - Should receive no more than 3 lines of systemic therapies in metastatic setting;
   - Radiographic progression during or after the most recent treatment.
6. For Part 2 HNSCC participants:
   - Histologically or cytologically confirmed locally advanced or metastatic HNSCC;
   - 2L+ HNSCC who have progressed after at least 1 line of standard of care systemic chemotherapy and one line of checkpoint inhibitors in the metastatic setting;
   - Should receive no more than 3 lines of systemic therapies in metastatic setting;
   - Radiographic progression during or after the most recent treatment.
7. A mandatory archived formalin fixed- paraffin embedded (FFPE) tumor tissue block must be provided. If an archived FFPE tissue is not available, a de novo (i.e., fresh) tumor sample should be obtained in accordance with local institutional practice for tumor biopsies.
8. Participants must have at least 1 measurable lesion (not previously irradiated) as defined by Response Evaluation Criteria in Solid Tumors that has not been previously irradiated.
9. Eastern Cooperative Oncology Group (ECOG) Performance Status (PS) 0 or 1.
10. Adequate Bone Marrow Function, including:
   a. Absolute Neutrophil Count (ANC) ≥1,500/mm³ or ≥1.5 x 10⁹/L;
   b. Platelets ≥100,000/mm³ or ≥100 x 10⁹/L;
   c. Hemoglobin ≥9 g/dL. Limited transfusions to reach this value are allowed, after discussion with the sponsor's medical monitor. There should not be a chronic need for transfusions in the recent (approximately 3 month) past.
11. Adequate Renal Function, including
   a. Serum creatinine ≥1.5 x upper limit of normal (ULN) or Estimated creatinine clearance ≥50 mL/min as calculated using the method standard for the institution. In equivocal cases, a 24-hour urine collection test can be used to estimate the creatinine clearance more accurately;
   b. Estimated creatinine clearance ≥40 mL/min for participants with urothelial carcinoma.
12. Adequate Liver Function, including:
   For Part 1A, 2A, 2B and 2C monotherapy
      a. Total serum bilirubin ≥1.5 x upper limit of normal (ULN) for Compound 1 monotherapy (unless the participant has documented Gilbert syndrome);
      b. Aspartate and Alanine aminotransferase (AST and ALT) ≤2.5 x ULN; ≤5.0 x ULN if there is liver involvement by the tumor) for Compound 1 monotherapy; AST and ALT ≥1.5 x ULN for Compound 1 in combination with docetaxel; For Part 1B and 2D in combination with Docetaxel
      c. Total bilirubin ≤ ULN for Compound 1 in combination with docetaxel;
      d. Aspartate and Alanine aminotransferase (AST and ALT) ≥1.5 x ULN; alkaline phosphatase (ALP) ≤2.5 x ULN for Compound 1 in combination with docetaxel.
13. Resolved acute effects of any prior therapy to baseline severity or CTCAE Grade ≤1 except for AEs not constituting a safety risk by investigator judgment. Participants with hypothyroidism of hypopituitarism resulting from immunotherapy on stable hormone replacement therapy and participants with adrenal insufficiency receiving doses <15 mg/day of prednisone equivalents are eligible.
14. Participants who are willing and able to comply with all scheduled visits, treatment plan, laboratory tests, lifestyle considerations, and other study procedures.
15. Capable of giving signed informed consent as described, which includes compliance with the requirements and restrictions listed in the informed consent document (ICD) and in this protocol.

### Dosage Form(s) and Packaging

Compound 1 is provided as tablets for oral administration. The 0.5 mg, 1 mg, and 5 mg are supplied in separate bottles and labeled according to local regulatory requirements.

Docetaxel is commercially available and is used in accordance with the US Package Insert (USPI).

### Administration

Participants swallows the investigational product whole, and do not manipulate (e.g., crush) or chew the investigational product prior to swallowing. Participants who are unable to swallow whole tablets may dissolve their dose in oral syringe(s) to allow for suspension administration either orally or via an NG (nasogastric) tube.

Compound 1 is administered orally initially QD, then BID or QD for subsequent cohorts on a continuous basis. If supported by emerging data, alternative intermittent dosing regimen may be considered. For monotherapy cohorts, a cycle is defined as continuous dosing of 28 days, regardless of missed doses or dose delays. For Cohorts 1B and 2D (Compound 1 in combination with docetaxel) a cycle is defined as 21 days to be consistent with docetaxel treatment cycles and dosing schedule.

For the BID dosing regimen, on days where the morning dose of Compound 1 is held due to a specific study visit, the second dose on that day should be taken no sooner than 8 hours, and no later than 16 hours post the first dose on that day (allowable treatment window ±4 hours). For once daily dosing regimen, the allowable treatment window is ±4 hours.

Compound 1 is administered orally on an empty stomach without adjustment for body size at every cycle. Participants are instructed to take their medication at approximately the same time each day and to not take more than the prescribed dose at any time. If a participant misses a dose or day of treatment, they must be instructed not to "make it up" but to resume subsequent doses as prescribed. In addition, if a participant vomits any time after taking a dose; they must be instructed not to "make it up" but to resume subsequent doses as prescribed. Lastly, if a participant inadvertently takes 1 extra dose during a day, the participant should not take the next dose of Compound 1.

Compound 1 tablets can also be dissolved into a suspension which can be administrated orally or via nasogastric (NG) tube if needed.

On Cycle 1 Day 15 and Day 16, Part 2 participants participating in the food effect assessment will receive Compound 1 under fasted and fed conditions, respectively, at the site, and have blood samples collected for PK characterization.

### Docetaxel Administration

For participants in Parts 1B and 2D, docetaxel is administered at 75 mg/m² intravenously over 1 hour on Day 1 of each 21 day cycle until disease progression, unacceptable toxicity, or participant refusal, whichever occurs first. All participants should be premedicated with oral corticosteroids such as dexamethasone 16 mg per day (e.g., 8 mg BID) for 3 days starting 1 day prior to docetaxel administration in order to reduce the incidence and severity of fluid retention as well as the severity of hypersensitivity reactions. Participants are observed closely for hypersensitivity reactions, especially during the first and second infusion.

### EXAMPLE 6

### QD Dosing of Patients with 4 mg, 6 mg, and 8 mg of Compound 1

Prior to administration of Compound 1 to patients in three cohorts (4 mg QD, 6 mg QD, and 8 mg QD), the investigator confirmed appropriate temperature and storage conditions had been maintained. Records were checked to ensure Compound 1 had been stored in a secure, environmentally controlled, and monitored area with limited access.

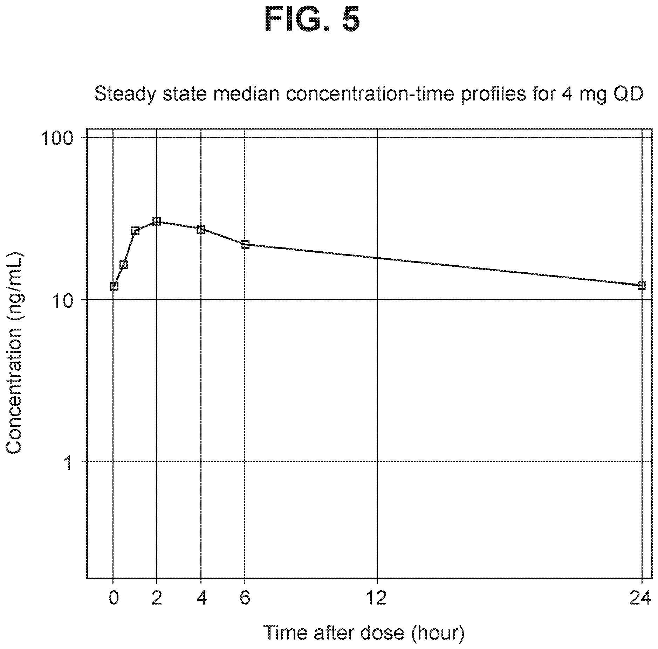

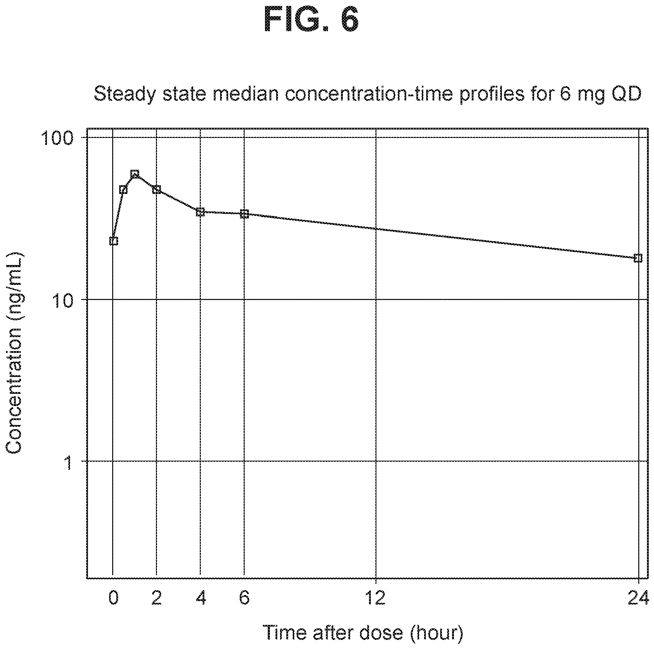

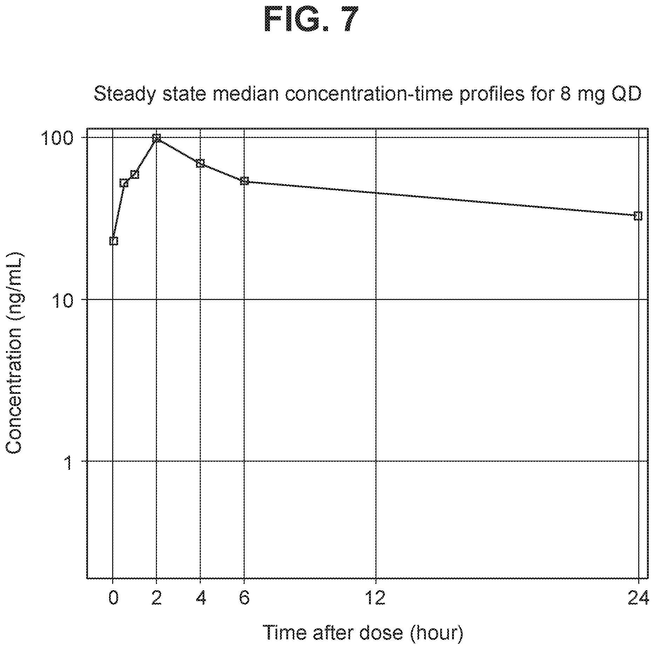

Compound 1 was provided to patients in three cohorts as tablets ((1) 1 mg tablets (for patient dosed with 4 mg QD); and 2) 1 mg and 5 mg tablets (for patients dosed with 6 mg QD and 8 mg QD)) supplied in separate labeled bottles for oral administration. Because patients were receiving 4 mg QD, 6 mg QD, and 8 mg QD, respectively, each of them were instructed to swallow whole (i.e., without crushing or chewing) 1) four 1 mg tablets (for the 4 mg QD cohort (N=3)); 2) one 5 mg tablets and one 1 mg tablet (for the 6 mg cohort (N=6)); and 3) one 5 mg tablet and three 1 mg tablets (for the 8 mg cohort (N=2)) at the same time each morning with at least 8 oz (240 mL) of water on an empty stomach. No food or liquids other than water were consumed for 2 hours before and 1 hour following each dose administration. Patients in these cohorts took Compound 1 orally QD as described above for four cycles (a cycle is 28 days). Weekly visits to the clinic were made by the patient to evaluate the anti-cancer activity and side effects of Compound 1. FIG. 5, FIG. 6, and FIG. 7 show the Day 15 steady state median concentration-time profiles for 4 mg QD, 6 mg QD, and 8 mg QD, respectively. Following QD dosing, low peak-to-trough ratios (median=3.1) were observed at steady state. Low peak to trough ratios are desirable because they help minimizing potential Cₘₐₓ-related adverse effects (if any) while maintaining the therapeutic effects. The low peak-to-trough ratios observed following QD dosing suggested that QD dosing regimen is feasible and also preferable for enhancing compliance and convenience among the cancer patients and their health care providers.

### EXAMPLE 7

### Blood Pharmacodynamic Biomarker SDMA Inhibition with Compound 1

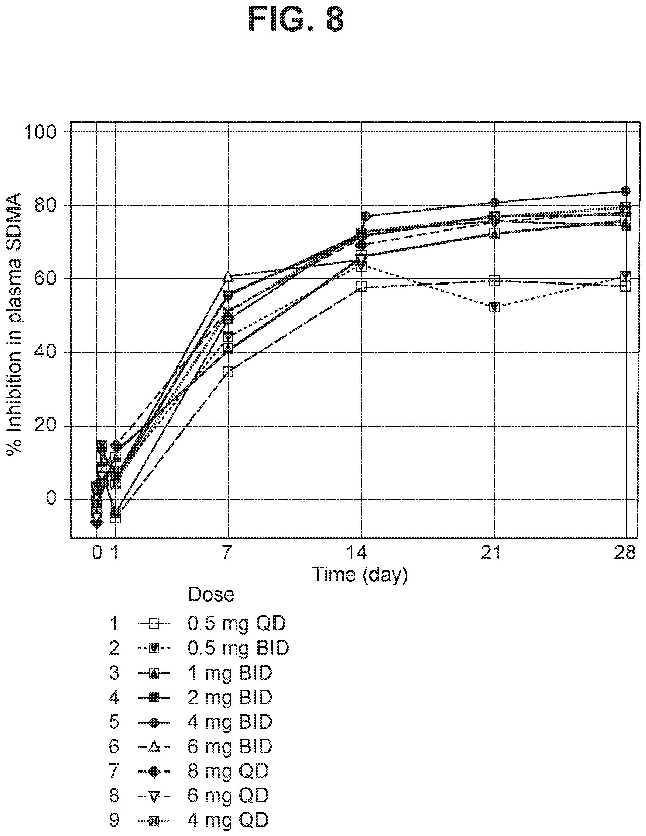

The plasma SDMA (Symmetrical Dimethylarginine) level was used as a pharmacodynamic biomarker (PD) to monitor the inhibition effect of Compound 1. SDMA was chosen for the blood PD biomarker because PRMT5 catalyzes the formation of SDMA in a number of nuclear and cytoplasmic proteins, and PRMT5 inhibition would result in a decrease of SDMA. The quantitative determination of SDMA was performed by using a liquid chromatography mass spectrometry (LC-MS/MS) assay which is an exceedingly sensitive and specific analytical technique that can precisely determine the identities and concentration of compounds such as SDMA within a sample. The assay had been validated from 1 - 500 ng/ml in plasma. FIG. 8 shows that different dose levels of Compound 1 reduced 60-80 % SDMA over time. The plasma SDMA inhibition were around 60% for the first two cohorts (N=1 in each cohort), including 0.5 mg QD and 0.5 mg BID. The plasma SDMA inhibition was around 80% from cohort 3 to cohort 9, including 1 mg BID (N=2), 2 mg BID (N=3), 4 mg BID (N=3), 6 mg BID (N=4), 4 mg QD (N=5), 6 mg QD (N=6), and 8 mg QD (N=3).

## Claims

1. Compound 1 which is (1S,2S,3S,5R)-3-((6-(difluoromethyl)-5-fluoro-1,2,3,4-tetrahydroisoquinolin-8-yl)oxy)-5-(4-methyl-7H-pyrrolo[2,3-d]pyrimidin-7-yl)cyclopentane-1,2-diol having the structure: 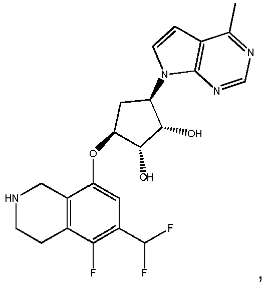 or a pharmaceutically acceptable salt thereof, for use in treating abnormal cell growth in a subject no more than once per day.

2. The Compound 1 for use according to claim 1, wherein said abnormal cell growth is cancer.

3. The Compound 1 for use according to Claim 2, wherein said cancer is selected from the group consisting of lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, and pituitary adenoma.

4. The Compound 1 for use according to any one of claims 1-3, wherein said Compound 1, or a pharmaceutically acceptable salt thereof, is administered in a dosage form selected from one or more tablets, one or more capsules, a liquid solution, a liquid suspension, or a syrup.

5. The Compound 1 for use according to any one of claims 1-4, wherein said administration takes place for seven consecutive days.

6. The Compound 1 for use according to any one of claims 1-4, wherein said administration takes place for one cycle comprising twenty-eight consecutive days.

7. The Compound 1 for use according to claim 6, wherein said administration takes place for 1 to 24 cycles.

8. The Compound 1 for use according to any one of claims 1-7, wherein said Compound 1 is administered to a subject at a dose of about 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 50 mg, 60 mg, 70 mg, 80 mg, 90 mg, 100 mg, 110 mg, 120 mg, 130 mg, 140 mg or 150 mg once daily.

9. The Compound 1 for use according to any one of claims 1-8, wherein said use further comprises administration of a second therapeutic agent.

10. The Compound 1 for use according to claim 9, wherein the second therapeutic agent is a chemotherapeutic agent.

11. The Compound 1 for use according to claim 9, wherein the second therapeutic agent is an antibody.

12. The Compound 1 for use according to any one of claims 9-11, wherein the second therapeutic agent is administered intravenously, subcutaneously or orally.

13. The Compound 1 for use according to any one of claims 1-10, wherein said use comprises premedication of the subject with a corticosteroid prior to the treatment with Compound 1.

14. The Compound 1 for use according to any one of claims 2-13, wherein the cancer is relapsed, refractory, or metastatic.

15. A medicament comprising a therapeutically effective amount of Compound 1 which is (1S,2S,3S,5R)-3-((6-(difluoromethyl)-5-fluoro-1,2,3,4-tetrahydroisoquinolin-8-yl)oxy)-5-(4-methyl-7H-pyrrolo[2,3-d]pyrimidin-7-yl)cyclopentane-1,2-diol having the structure: 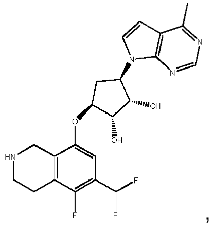 or a pharmaceutically acceptable salt thereof, for use in treating abnormal cell growth in a subject no more than once per day.

16. A medicament for use according to claim 15 wherein said medicament further comprises an effective amount of a second therapeutic agent.

## Patentansprüche

1. Verbindung 1, die (1S,2S,3S,5R)-3-((6-(Difluormethyl)-5-fluor-1,2,3,4- tetrahydroisochinolin-8-yl)oxy)-5-(4-methyl-7H-pyrrolo[2,3-d]pyrimidin-7-yl)cyclopentan-1,2-diol mit der folgenden Struktur ist: 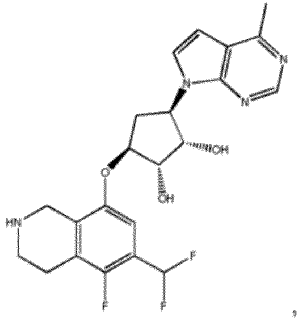 oder ein pharmazeutisch annehmbares Salz davon, zur Verwendung bei der Behandlung von abnormalem Zellwachstum bei einem Subjekt nicht mehr als einmal pro Tag.

2. Verbindung 1 zur Verwendung nach Anspruch 1, wobei das abnormale Zellwachstum Krebs ist.

3. Verbindung 1 zur Verwendung nach Anspruch 2, wobei der Krebs ausgewählt ist aus der Gruppe bestehend aus Lungenkrebs, Knochenkrebs, Bauchspeicheldrüsenkrebs, Hautkrebs, Kopf- oder Halskrebs, kutanem oder intraokularem Melanom, Gebärmutterkrebs, Eierstockkrebs, Rektalkrebs, Krebs der Analregion, Magenkrebs, Dickdarmkrebs, Brustkrebs, Gebärmutterkrebs, Eileiterkrebs, Gebärmutterschleimhautkrebs, Gebärmutterhalskrebs, Vaginakrebs, Vulvakrebs, Morbus Hodgkin, Speiseröhrenkrebs, Dünndarmkrebs, Krebs des endokrinen Systems, Schilddrüsenkrebs, Nebenschilddrüsenkrebs, Nebennierenkrebs, Weichteilsarkom, Krebs der Harnröhre, Peniskrebs, Prostatakrebs, chronischer oder akuter Leukämie, lymphozytären Lymphomen, Blasenkrebs, Nieren- oder Harnleiterkrebs, Nierenzellkarzinom, Nierenbeckenkrebs, Neubildungen des Zentralnervensystems (ZNS), primären ZNS-Lymphomen, Wirbelsäulentumoren, Hirnstamm-Gliomen und Hypophysenadenomen.

4. Verbindung 1 zur Verwendung nach einem der Ansprüche 1 bis 3, wobei die Verbindung 1 oder ein pharmazeutisch akzeptables Salz davon in einer Dosierungsform verabreicht wird, die aus einer oder mehreren Tabletten, einer oder mehreren Kapseln, einer flüssigen Lösung, einer flüssigen Suspension oder einem Sirup ausgewählt ist.

5. Verbindung 1 zur Verwendung nach einem der Ansprüche 1-4, wobei die Verabreichung an sieben aufeinanderfolgenden Tagen erfolgt.

6. Verbindung 1 zur Verwendung nach einem der Ansprüche 1 bis 4, wobei die Verabreichung über einen Zyklus erfolgt, der achtundzwanzig aufeinanderfolgende Tage umfasst.

7. Verbindung 1 zur Verwendung nach Anspruch 6, wobei die Verabreichung über 1 bis 24 Zyklen erfolgt.

8. Verbindung 1 zur Verwendung nach einem der Ansprüche 1 bis 7, wobei die Verbindung 1 einem Subjekt in einer Dosis von etwa 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 50 mg, 60 mg, 70 mg, 80 mg, 90 mg, 100 mg, 110 mg, 120 mg, 130 mg, 140 mg oder 150 mg einmal täglich verabreicht wird.

9. Verbindung 1 zur Verwendung nach einem der Ansprüche 1-8, wobei die Verwendung ferner die Verabreichung eines zweiten therapeutischen Mittels umfasst.

10. Verbindung 1 zur Verwendung nach Anspruch 9, wobei das zweite therapeutische Mittel ein chemotherapeutisches Mittel ist.

11. Verbindung 1 zur Verwendung nach Anspruch 9, wobei das zweite therapeutische Mittel ein Antikörper ist.

12. Verbindung 1 zur Verwendung nach einem der Ansprüche 9 bis 11, wobei das zweite therapeutische Mittel intravenös, subkutan oder oral verabreicht wird.

13. Verbindung 1 zur Verwendung nach einem der Ansprüche 1-10, wobei die Verwendung die Prämedikation des Subjekts mit einem Kortikosteroid vor der Behandlung mit der Verbindung 1 umfasst.

14. Verbindung 1 zur Verwendung nach einem der Ansprüche 2-13, wobei der Krebs rezidiviert, refraktär oder metastasiert ist.

15. Medikament, umfassend eine therapeutisch wirksame Menge der Verbindung 1, die (1S,2S,3S,5R)-3-((6-(Difluormethyl)-5-fluor-1,2,3,4-tetrahydroisochinolin-8-yl)oxy)-5-(4-Methyl-7H-pyrrolo[2,3-d]pyrimidin-7-yl)cyclopentan-1,2-diol mit der folgenden Struktur ist: 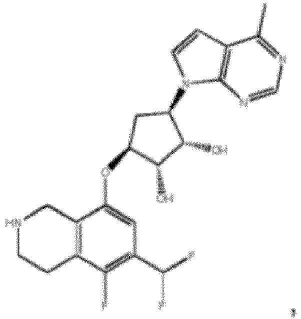 oder ein pharmazeutisch annehmbares Salz davon, zur Verwendung bei der Behandlung von abnormalem Zellwachstum bei einem Subjekt nicht mehr als einmal pro Tag.

16. Medikament zur Verwendung gemäß Anspruch 15, wobei das Medikament weiterhin eine wirksame Menge eines zweiten therapeutischen Mittels umfasst.

## Revendications

1. Composé 1 qui est le (1S,2S,3S,5R)-3-((6-(difluorométhyl)-5-fluoro-1,2,3,4-tétrahydroisoquinoléin-8-yl)oxy)-5-(4-méthyl-7H-pyrrolo[2,3-d]pyrimidin-7-yl)cyclopentane-1,2-diol ayant la structure : 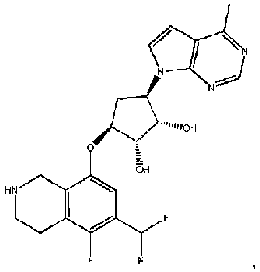 ou sel pharmaceutiquement acceptable de celui-ci, pour son utilisation dans le traitement d'une croissance cellulaire anormale chez un sujet pas plus d'une fois par jour.

2. Composé 1 pour son utilisation selon la revendication 1, dans lequel ladite croissance cellulaire anormale est un cancer.

3. Composé 1 pour son utilisation selon la revendication 2, dans lequel ledit cancer est sélectionné parmi le groupe constitué d'un cancer du poumon, d'un cancer des os, d'un cancer du pancréas, d'un cancer de la peau, d'un cancer de la tête ou du cou, d'un mélanome cutané ou intraoculaire, d'un cancer de l'utérus, d'un cancer de l'ovaire, d'un cancer rectal, d'un cancer de la région anale, d'un cancer de l'estomac, d'un cancer du côlon, d'un cancer du sein, d'un cancer de l'utérus, d'un carcinome des trompes de Fallope, d'un carcinome de l'endomètre, d'un carcinome du col de l'utérus, d'un carcinome du vagin, d'un carcinome de la vulve, de la maladie d'Hodgkin, d'un cancer de l'œsophage, d'un cancer de l'intestin grêle, d'un cancer du système endocrinien, d'un cancer de la glande thyroïde, d'un cancer de la glande parathyroïde, d'un cancer de la glande surrénale, d'un sarcome des tissus mous, d'un cancer de l'urèthre, d'un cancer du pénis, d'un cancer de la prostate, d'une leucémie chronique ou aiguë, de lymphomes lymphocytaires, d'un cancer de la vessie, d'un cancer du rein ou de l'uretère, d'un carcinome des cellules rénales, d'un carcinome du bassinet rénal, de néoplasmes du système nerveux central (SNC), d'un lymphome primaire du SNC, de tumeurs de l'axe spinal, d'une tumeur du tronc cérébral, et d'un adénome pituitaire.

4. Composé 1 pour son utilisation selon l'une des revendications 1 à 3, dans lequel ledit composé 1, ou un sel pharmaceutiquement acceptable de celui-ci, est administré en une forme galénique sélectionnée parmi un ou plusieurs comprimés, une ou plusieurs capsules, une solution liquide, une suspension liquide, ou un sirop.

5. Composé 1 pour son utilisation selon l'une des revendications 1 à 4, dans lequel ladite administration a lieu pendant sept jours consécutifs.

6. Composé 1 pour son utilisation selon l'une des revendications 1 à 4, dans lequel ladite administration a lieu pendant un cycle comprenant vingt-huit jours consécutifs.

7. Composé 1 pour son utilisation selon la revendication 6, dans lequel ladite administration a lieu pendant 1 à 24 cycles.

8. Composé 1 pour son utilisation selon l'une des revendications 1 à 7, dans lequel ledit composé 1 est administré à un sujet en une dose d'environ 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 50 mg, 60 mg, 70 mg, 80 mg, 90 mg, 100 mg, 110 mg, 120 mg, 130 mg, 140 mg, ou 150 mg une fois par jour.

9. Composé 1 pour son utilisation selon l'une des revendications 1 à 8, dans lequel ladite utilisation comprend en outre l'administration d'un deuxième agent thérapeutique.

10. Composé 1 pour son utilisation selon la revendication 9, dans lequel le deuxième agent thérapeutique est un agent chimiothérapeutique.

11. Composé 1 pour son utilisation selon la revendication 9, dans lequel le deuxième agent thérapeutique est un anticorps.

12. Composé 1 pour son utilisation selon l'une des revendications 9 à 11, dans lequel le deuxième agent thérapeutique est administré par voie intraveineuse, sous-cutanée ou orale.

13. Composé 1 pour son utilisation selon l'une des revendications 1 à 10, dans lequel ladite utilisation comprend une prémédication du sujet avec un corticoïde avant le traitement avec le composé 1.

14. Composé 1 pour son utilisation selon l'une des revendications 2 à 13, dans lequel le cancer est en rechute, réfractaire ou métastasique.

15. Médicament comprenant une quantité thérapeutiquement efficace du composé 1 qui est le (1S,2S,3S,5R)-3-( (6-(difluorométhyl)-5-fluoro-1,2,3,4-tétrahydroisoquinoléin-8-yl)oxy)-5-(4-méthyl-7H-pyrrolo[2,3-d]pyrimidin-7-yl)cyclopentane-1,2-diol ayant la structure : 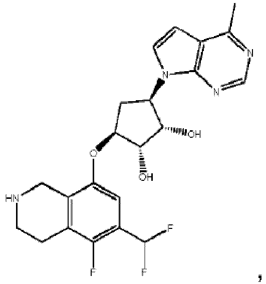 ou un sel pharmaceutiquement acceptable de celui-ci, pour son utilisation dans le traitement d'une croissance cellulaire anormale chez un sujet pas plus d'une fois par jour.

16. Médicament pour son utilisation selon la revendication 15 dans lequel ledit médicament comprend en outre une quantité efficace d'un deuxième agent thérapeutique.
